(19) Europäisches Patentamt
European Patent Office
Office européen des brevets

(11) **EP 4 032 517 A1**

(12) **EUROPEAN PATENT APPLICATION**
published in accordance with Art. 153(4) EPC

(43) Date of publication:
**27.07.2022 Bulletin 2022/30**

(21) Application number: **19951176.7**

(22) Date of filing: **20.11.2019**

(51) International Patent Classification (IPC):
***A61F 13/49*** *(2006.01)*

(52) Cooperative Patent Classification (CPC):
**A61F 13/49; A61F 13/51; A61F 13/514**

(86) International application number:
**PCT/JP2019/045424**

(87) International publication number:
**WO 2021/084764 (06.05.2021 Gazette 2021/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.11.2019 CN 201911059379**

(71) Applicant: **Daio Paper Corporation**
**Ehime 799-0492 (JP)**

(72) Inventor: **MINAMI, Takeshi**
**Nantong, Jiangsu 226010 (CN)**

(74) Representative: **Held, Stephan**
**Meissner Bolte Patentanwälte**
**Rechtsanwälte Partnerschaft mbB**
**Widenmayerstraße 47**
**80538 München (DE)**

(54) **STRETCHABLE MEMBER AND DISPOSABLE WEARABLE ARTICLE HAVING SAID STRETCHABLE MEMBER**

(57)    To provide a stretchable member having vent holes in an elastic sheet and having an excellent aesthetic appearance.

The problem can be solved by a stretchable member in which an elastic sheet 30 is interposed between a second sheet layer 20B having an exposed portion and a first sheet layer 20A, the second sheet layer 20B and the first sheet layer 20A are bonded to each other at a large number of sheet bonding portions 40 arranged at intervals through bonding holes 31 penetrating the elastic sheet 30 or via the elastic sheet 30, vent holes 33 are opened by separation of edges of the bonding holes 31 from outer peripheral edges of the sheet bonding portions 40 in a stretchable direction ED at least in an unfolded state, a color of an outer surface of the elastic sheet and a color of a portion of an outer surface of the first sheet layer 20A seen from the vent holes 33 are see-through through the second sheet layer 20B, and a color difference ΔE between a color of an outer surface 30s of the elastic sheet 30 and a color of an outer surface 20s of the first sheet layer 20A is 5 or less.

[FIG.16]

**Description**

Technical Field

The present invention relates to a stretchable member having a stretchable structure in which an elastic sheet such as an elastic film is sandwiched between a first sheet layer and a second sheet layer, and a disposable wearing article including the stretchable member.

Background Art

In a disposable wearing article such as a disposable diaper, in order to improve fitting to a body surface, elasticity is generally imparted to an appropriate place such as a periphery of a leg or a lower torso. As a technique for imparting elasticity, conventionally, a technique for attaching an elongated elastic member such as a rubber thread in a state in which the elongated elastic member is stretched in a longitudinal direction thereof has been widely adopted. However, when it is desired to impart elasticity with a certain width, an aspect is adopted in which rubber threads are fixed in a state of being arranged side by side at intervals in a width. As a technique having excellent surface fitting, a technique for attaching an elastic sheet in a state where the elastic sheet is stretched in an elasticity imparting direction has also been proposed. (See, for example, Patent Literatures 1 and 2) .

In the stretchable member including the elastic sheet, the elastic sheet is stacked between the first sheet layer and the second sheet layer, and in a state in which the elastic sheet is stretched in a stretchable direction, the first sheet layer and the second sheet layer are bonded to each other through bonding holes formed in the elastic sheet at a large number of dotted sheet bonding portions arranged at intervals in the stretchable direction and a direction orthogonal thereto. In addition, in the stretchable member, in a natural length state, as the elastic sheet is contracted between the sheet bonding portions, an interval between the sheet bonding portions becomes narrower, and pleats extending in a direction crossing the stretchable direction are formed between the sheet bonding portions in the first sheet layer and the second sheet layer. On the contrary, at the time of stretch, as the elastic sheet is stretched between the sheet bonding portions, an interval between the sheet bonding portions and pleats in the first sheet layer and the second sheet layer become wider, and elastic stretch is possible until the first sheet layer and the second sheet layer are fully unfolded. In a state where the elastic sheet is stretched at least to some extent, edges of the bonding holes are separated from outer peripheral edges of the sheet bonding portions in the stretchable direction, whereby the vent holes are opened.

On the other hand, since the disposable wearing article is used together with or as a substitute for underwear, the stretchable member used for the disposable wearing article is required not only to meet functional requirements such as air permeability, fitting, and flexibility, but also to have an appearance close to cloth. Therefore, a nonwoven fabric which is a thin and flexible material is suitable as the first sheet layer and the second sheet layer.

However, in the present elastic sheet stretchable structure, when the first sheet layer or the second sheet layer is a sheet having a certain degree of translucency such as a nonwoven fabric, the shapes of the vent holes are slightly seen through. Therefore, there is a possibility that a user may feel that the appearance is not so preferable.

Citation List

Patent Literature

Patent Literature 1: JP 2016-189932 A
Patent Literature 2: JP 2015-204982 A
Patent Literature 3: JP 2016-140477 A
Patent Literature 4: JP 2016-189931 A
Patent Literature 5: JP 2016-189933 A
Patent Literature 6: JP 2017-064224 A
Patent Literature 7: JP 2017-148169 A
Patent Literature 8: JP 2017-225508 A

Summary of Invention

Technical Problem

Therefore, a main object of the present invention is to provide a stretchable member having an excellent aesthetic appearance while having vent holes in an elastic sheet.

Solution to Problem

A stretchable member that has solved the above problems and a disposable wearing article including the stretchable member are as follows.

<First Aspect>

A stretchable member having an elastic sheet stretchable structure, the elastic sheet stretchable structure including an outer sheet layer having an exposed portion, a lower sheet layer, and an elastic sheet interposed between the outer sheet layer and the lower sheet layer, the outer sheet layer and the lower sheet layer being bonded to each other at a large number of sheet bonding portions arranged at intervals through bonding holes penetrating the elastic sheet or via the elastic sheet, wherein

a region having the elastic sheet stretchable structure has a stretchable region that is contracted in a stretchable direction by contraction of the elastic sheet and is extensible in the stretchable direction,
vent holes are opened by separation of edges of the bonding holes from outer peripheral edges of the sheet bonding portions in the stretchable direction at least in an unfolded state,
a color of an outer surface of the elastic sheet and a color of a portion of an outer surface of the lower sheet layer seen from the vent holes are see-through through the outer sheet layer, and
a color difference ΔE between the color of the outer surface of the elastic sheet and the color of the outer surface of the lower sheet layer is 5 or less.

(Action and effect)

If it is only desired to make the shapes of the vent holes difficult to see, it is only required to make the elastic sheet transparent. However, there is a case where it is not preferable to enhance the transparency of the elastic sheet due to circumstances such as the characteristics and price of the elastic sheet. On the other hand, as in the present stretchable member, when the color difference ΔE between the color of the outer surface of the elastic sheet and the color of the outer surface of the lower sheet layer is 5 or less, the difference between the color of the outer surface of the elastic sheet and the color of the portion of the outer surface of the lower sheet layer seen from the vent holes is small. Therefore, it is difficult to visually recognize the shapes of the vent holes through the outer sheet layer. As a result, the present stretchable member has an excellent aesthetic appearance.

<Second Aspect>

The stretchable member recited in the first aspect, wherein
the elastic sheet has a light transmittance of 40 to 60%.

(Action and effect)

As described above, if it is only desired to make the shapes of the vent holes difficult to see, the higher the light transmittance of the elastic sheet is, the better. However, within the above-described range of the color difference, even when the light transmittance of the elastic sheet is low, that is, even within the range of the present aspect, the shapes of the vent holes can be made difficult to see.

<Third Aspect>

The stretchable member recited in the first or second aspect, wherein
the light transmittance of the lower sheet layer is 0.5 to 2.0 times the light transmittance of the elastic sheet.

(Action and effect)

When a difference in light transmittance between the lower sheet layer and the elastic sheet is large, the shapes of the vent holes are easily visible due to shadow. Therefore, the lower sheet layer and the elastic sheet preferably have a light transmittance within the range of the present aspect.

<Fourth Aspect>

The stretchable member recited in any one of the first to third aspects, wherein

the outer sheet layer is a perforated nonwoven fabric having holes arranged at intervals and penetrating front and back surfaces of the nonwoven fabric, and
a color difference ∆E between a color of an outer surface of the elastic sheet and a color of an outer surface of the outer sheet layer is 5 or less.

(Action and effect)

Since the outer sheet layer uniformly covers an outer side of the elastic sheet, even if the color difference from the elastic sheet is large, an appearance of the vent holes is not directly affected. However, when a perforated nonwoven fabric is used for the outer sheet layer in order to enhance air permeability of the outer sheet layer, the holes of the perforated nonwoven fabric are conspicuous, and there is a possibility that a user may feel that the appearance is not so preferable. On the other hand, as in the present aspect, when the color difference ∆E between the color of the outer surface of the elastic sheet and the color of the outer surface of the outer sheet layer is 5 or less, the difference between the color of the outer surface of the elastic sheet and the color of the outer surface of the outer sheet layer is small. Therefore, the shapes of the holes of the outer sheet layer are less likely to be conspicuous. As a result, the present stretchable member has an excellent aesthetic appearance while improving air permeability using a perforated nonwoven fabric for the outer sheet layer.

<Fifth Aspect>

The stretchable member recited in any one of the first to fourth aspects, wherein
each of the outer sheet layer, the elastic sheet, and the lower sheet layer has an outer surface having a whiteness of 60 to 80%.

(Action and effect)

Visibility of the holes varies depending on the degree of shadow generated at edges of the holes. When the whiteness is 80% or less, the whiteness of a portion outside the holes is low, and therefore a difference between the portion outside the holes and the shadow generated at the edges of the holes is reduced, and the visibility of the holes is reduced. Therefore, in order to reduce the visibility of the holes, the whiteness of a material is preferably low. However, since the disposable wearing article is a sanitary product, white tends to be preferred. Therefore, each of the sheet layers and the elastic sheet preferably have a whiteness within the range of the present aspect.

<Sixth Aspect>

The stretchable member recited in any one of the first to fifth aspects, wherein
the outer sheet layer is a nonwoven fabric having a constituent fiber fineness of 1.0 to 6.0 dtex and a basis weight of 15 to 25 g/m$^2$.

(Action and effect)

In consideration of texture of an outer surface of a product, the outer sheet layer is preferably a nonwoven fabric within the scope of the present aspect. However, such a nonwoven fabric has a high light transmittance (for example, 50% or more), and therefore vent holes are easily seen through. Therefore, the above-described color difference is particularly significant when being applied to a case having the outer sheet layer as in the present aspect.

<Seventh Aspect>

The stretchable member recited in any one of the first to sixth aspects, wherein
the vent holes are opened in the stretchable region in a natural length state.

(Action and effect)

Since the vent holes are formed by separation of edges of the bonding holes from outer peripheral edges of the sheet bonding portions in the stretchable direction, the vent holes are deformed by elongation of the elastic sheet, and become larger as approaching an unfolded state. Depending on the shape (for example, circular shape) of the sheet bonded portion, in a natural length state, the vent holes are not necessarily formed because of close contact between the edges of the bonding holes and the outer peripheral edges of the sheet bonding portions. Even in this case, in a state where the elastic sheet is stretched to some extent such as a wearing state, the vent holes are opened at least on both sides of the sheet bonding portions in the stretchable direction because the bonding holes are stretched in the stretchable direction. Of course, in order to exhibit good air permeability regardless of a stretched state, in the stretchable region, the vent holes are preferably opened in a natural length state as in the present stretchable member. However, since this type of product is sold in a natural length state, if the elastic sheet has an opening in the natural length state, an appearance of the product before use may be affected. Therefore, each of the aspects described above is particularly significant when being applied to a structure in which vent holes are opened in a natural length state.

<Eighth Aspect>

An underpants-type disposable wearing article including: an integrated outer member from a front body to a back body or outer members separately provided on the front body and the back body; an inner member attached to an intermediate portion of the outer member in a width direction and extending over both front and back sides of a crotch portion; a side seal portion in which both side portions of the outer member in the front body and both side portions of the outer member in the back body are bonded to each other; and a waist opening and a pair of left and right leg openings, wherein
the outer member in at least one of the front body and the back body is a stretchable member including the elastic sheet stretchable structure recited in any one of the first to seventh aspects over a width direction range corresponding to a portion between the side seal portions in at least a partial range in the front-back direction such that a stretchable direction of the stretchable region is the width direction.

(Action and effect)

As in the present aspect, the above-described stretchable member is suitable for an outer member of an underpants-type disposable wearing article.

Advantageous Effects of Invention

The present invention brings about, for example, an advantage that a stretchable member has an excellent aesthetic appearance while having vent holes in an elastic sheet.

Brief Description of Drawings

Fig. 1 is a plan view (internal surface side) of an underpants-type disposable diaper in an unfolded state.
Fig. 2 is a plan view (external surface side) of an underpants-type disposable diaper in an unfolded state.
Fig. 3 is a plan view illustrating only a main part of an underpants-type disposable diaper in an unfolded state.
Fig. 4(a) is a cross-sectional view taken along line C-C in Fig. 1, and Fig. 4(b) is a cross-sectional view taken along line E-E in Fig. 1.
Fig. 5 is a cross-sectional view taken along line A-A in Fig. 1.
Fig. 6 is a cross-sectional view taken along line B-B in Fig. 1.
Fig. 7 is a plan view (external surface side) of an underpants-type disposable diaper in an unfolded state.
Fig. 8(a) is a cross-sectional view taken along line C-C in Fig. 7, and Fig. 8(b) is a cross-sectional view taken along line E-E in Fig. 7.
Fig. 9(a) is a plan view of a main part of a stretchable region, Fig. 9(b) is a cross-sectional view taken along line D-D in Fig. 9(a), Fig. 9(c) is a cross-sectional view in a wearing state, and Fig. 9(d) is a cross-sectional view in a natural length state.
Fig. 10 is a plan view illustrating various shapes of sheet bonding portions.
Fig. 11 is a plan view of a stretchable region in an unfolded state.
Fig. 12 is an enlarged plan view illustrating a main part of a stretchable region in an unfolded state.
Fig. 13 is an enlarged plan view illustrating a main part of a stretchable region in a natural length state.
Fig. 14(a) is a cross-sectional view taken along line D-D in Fig. 12, and Fig. 14(b) is a cross-sectional view in a natural length state.

Fig. 15 is a plan view of a stretchable region in an unfolded state.

Fig. 16 is an enlarged plan view illustrating a main part of a stretchable region in an unfolded state.

Fig. 17 is an enlarged plan view illustrating a main part of a stretchable region in a natural length state.

Fig. 18 is a cross-sectional view schematically illustrating a cross-section of a main part of an outer member that has been stretched to some extent.

Fig. 19 is a cross-sectional view schematically illustrating a cross-section of a main part of an outer member that has been stretched to some extent.

Fig. 20 is a schematic view of an ultrasonic sealing device.

Fig. 21(a) is a plan view of a main part of a non-stretchable region, Fig. 21(b) is a cross-sectional view taken along line D-D in Fig. 21(a), Fig. 21(c) is a cross-sectional view in a wearing state, and Fig. 21(d) is a cross-sectional view in a natural length state.

Fig. 22 is a plan view of a main part of a non-stretchable region.

Fig. 23 is an enlarged plan view of a main part illustrating various patterns of holes of a perforated nonwoven fabric.

Fig. 24 is an enlarged plan view of a main part illustrating various patterns of holes of a perforated nonwoven fabric.

Fig. 25 is an enlarged plan view of a main part illustrating a chain pattern of holes of a perforated nonwoven fabric.

Fig. 26 illustrates various cross-sectional views of a perforated nonwoven fabric.

Description of Embodiments

Hereinafter, an example of a disposable wearing article will be described in detail with reference to the attached drawings. Note that a dotted pattern portion in the drawings indicates an adhesive as a bonding means for bonding constituent members located on a front surface side of the dotted pattern portion and a back surface side thereof, and is formed by applying a hot melt adhesive by solid application, bead application, curtain application, summit application, spiral application, pattern coating (transfer of a hot melt adhesive by a letterpress method), or the like. A fixing portion of an elastic member is formed, instead of this or in addition to this, by application to an outer peripheral surface of an elastic member by a comb gun, SureWrap application, or the like. Examples of the hot melt adhesive include an EVA-based agent, a pressure sensitive adhesive rubber-based agent (elastomer-based agent), a polyolefin-based agent, and a polyester/polyamide-based agent, and these can be used without particular limitation. As a bonding means for bonding constituent members, a means by material welding such as heat sealing or ultrasonic sealing can also be used.

As a nonwoven fabric in the following description, a known nonwoven fabric can be appropriately used according to a site or a purpose. Examples of a constituent fiber of the nonwoven fabric include, but are not limited to, a synthetic fiber such as a polyolefin-based fiber including polyethylene and polypropylene, a polyester-based fiber, or a polyamide-based fiber (including a composite fiber such as core-sheath in addition to a single component fiber), a regenerated fiber such as rayon or cupra, and a natural fiber such as cotton. These fibers can be mixed and used. In order to enhance flexibility of the nonwoven fabric, it is preferable to use a crimped fiber as the constituent fiber. The constituent fiber of the nonwoven fabric may be a hydrophilic fiber (including a hydrophilic fiber that has become hydrophilic by a hydrophilizing agent), a hydrophobic fiber, or a water-repellent fiber (including a water-repellent fiber that has become water-repellent by a water repellent agent). The nonwoven fabric is generally classified into a short fiber nonwoven fabric, a long fiber nonwoven fabric, a spunbonded nonwoven fabric, a meltblown nonwoven fabric, a spunlace nonwoven fabric, a thermal bond (air through) nonwoven fabric, a needle punch nonwoven fabric, a point bond nonwoven fabric, a stacked nonwoven fabric an (in addition to an SSS nonwoven fabric in which the same or similar nonwoven fabric layers are stacked, an SMS nonwoven fabric, an SMMS nonwoven fabric, or the like in which different nonwoven fabric layers are stacked and a meltblown layer is sandwiched between spunbond layers), and the like depending on a fiber length, a sheet forming method, a fiber bonding method, and a stacked structure, and any of these nonwoven fabrics can be used. The stacked nonwoven fabric means one that is manufactured as an integrated nonwoven fabric including all the layers and has been subjected to fiber bonding processing over all the layers, and does not include one obtained by bonding a plurality of separately manufactured nonwoven fabrics to each other by a bonding means such as a hot melt adhesive.

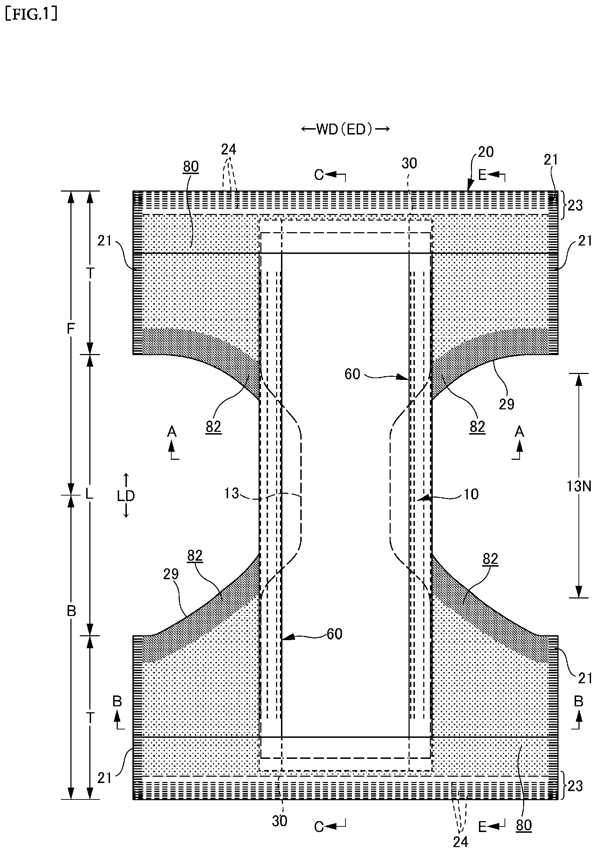

Figs. 1 to 6 illustrate an underpants-type disposable diaper. Reference character LD (longitudinal direction) represents a front-back direction, and reference character WD represents a width direction. This underpants-type disposable diaper (hereinafter also simply referred to as a diaper) includes an outer member 20 forming a front body F and a back body B, and an inner member 10 fixed to and integrated with an inner surface of the outer member 20. The inner member 10 is formed by interposing an absorber 13 between a liquid pervious top sheet 11 and a liquid impervious sheet 12. In manufacture, a back surface of the inner member 10 is bonded to an inner surface (upper surface) of the outer member 20 by a bonding means such as a hot melt adhesive. Thereafter, the inner member 10 and the outer member 20 are folded at the center in a front-back direction LD (longitudinal direction) that is a boundary between the front body F and the back body B. Both side portions thereof are bonded to each other by heat welding, a hot melt adhesive, or the like to form a side seal portion 21, thus forming an underpants-type disposable diaper with a waist opening and a pair of left and right leg openings.

(Structure example of inner member)

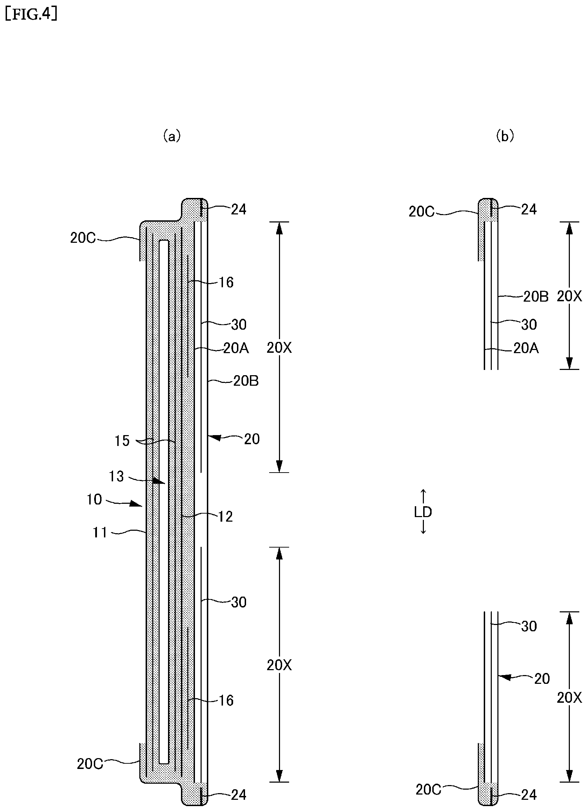

As illustrated in Figs. 4 to 6, the inner member 10 has a structure in which the absorber 13 is interposed between the top sheet 11 and the liquid impervious sheet 12 made of polyethylene or the like, and absorbs and holds an excretory liquid that has passed through the top sheet 11. The planar shape of the inner member 10 is not particularly limited, but is generally rectangular as illustrated in Fig. 1.

As the top sheet 11 covering a front surface side (skin side) of the absorber 13, a perforated nonwoven fabric (having holes formed by needle piercing, punching, or the like), an imperforate nonwoven fabric (having fiber gaps but not having holes formed by needle piercing or punching), a perforated plastic sheet, or the like is suitably used.

As the liquid impervious sheet 12 covering a back surface side (non-skin contact side) of the absorber 13, a liquid impervious plastic sheet such as polyethylene or polypropylene can be used. In particular, from a viewpoint of preventing stuffiness, a sheet having moisture permeability, for example, a microporous sheet obtained by melt-kneading an inorganic filler in a polyolefin resin such as polyethylene or polypropylene to form a sheet and then stretching the sheet in a uniaxial or biaxial direction can be suitably used.

As the absorber 13, a known absorber, for example, a pulp fiber stack, an assembly of filaments such as cellulose acetate, or a nonwoven fabric can be used. Alternatively, an absorber obtained by mixing or fixing a super absorbent polymer with the above absorber as necessary can be used. The absorber 13 can be wrapped by a wrapping sheet 15 having liquid perviousness and a liquid holding property, such as crepe paper, as necessary, for holding the shape and a polymer, for example.

The shape of the absorber 13 is formed into a substantially hourglass shape having a narrowing portion 13N narrower than both the front and back sides at a crotch portion. The size of the narrowing portion 13N can be determined appropriately, but the length of the narrowing portion 13N in the front-back direction can be about 20 to 50% of the maximum length of a diaper. The width of the narrowest portion can be about 40 to 60% of the maximum width of the absorber 13. In a case where such a narrowing portion 13N is included, if the planar shape of the inner member 10 is substantially rectangular, in a portion corresponding to the narrowing portion 13N of the absorber 13 in the inner member 10, a non-absorber side portion 17 including no absorber 13 is formed.

The liquid impervious sheet 12 is folded back to a back surface side on both sides of the absorber 13 in the width direction together with the top sheet 11. As the liquid impervious sheet 12, it is desirable to use an opaque sheet such that brown color of excreta, urine, or the like does not appear. For opacification, a film obtained by internally adding a pigment or a filler such as calcium carbonate, titanium oxide, zinc oxide, white carbon, clay, talc, or barium sulfate to a plastic is suitably used.

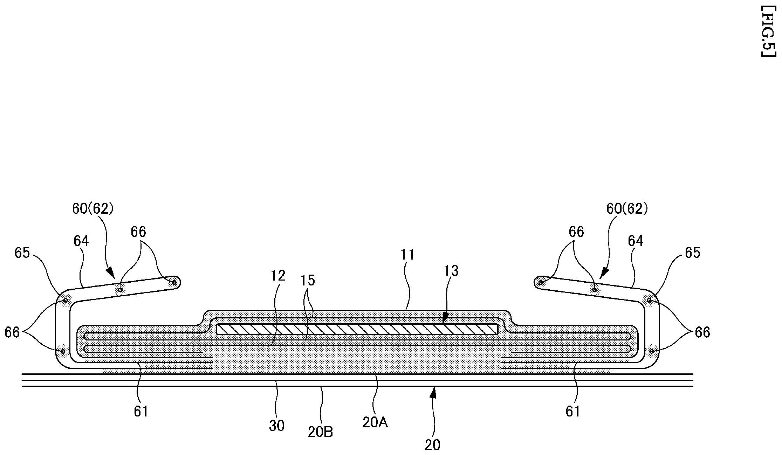

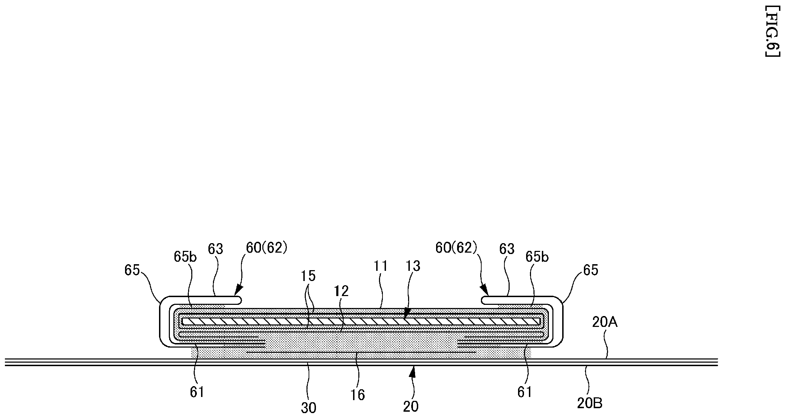

Rising gathers 60 that fit a periphery of a leg are formed on both sides of the inner member 10. As illustrated in Figs. 5 and 6, each of the rising gathers 60 includes a fixed portion 61 fixed to a side portion of a back surface of the inner member 10, a main unit portion 62 extending from the fixed portion 61 up to a side portion of a surface of the inner member 10 through a side of the inner member 10, a fallen portion 63 formed by fixing front and back end portions of the main unit portion 62 to a side portion of a surface of the inner member 10 (the top sheet 11 in the illustrated example) with a hot melt adhesive 65b or the like in a fallen state, and a free portion 64 formed by making a portion between the fallen portions 63 non-fixed. Each of these portions is formed of a gather sheet 65 that is a duplicate sheet obtained by folding back a sheet such as a nonwoven fabric. The gather sheet 65 is attached over the entire front-back direction of the inner member 10. The fallen portion 63 is disposed in front of the non-absorber side portion 17 and behind the non-absorber side portion 17. The free portion 64 extends to both the front and back sides of the non-absorber side portion 17. Between the duplicate gather sheets 65, a gather elastic member 66 is disposed at a tip of the free portion or the like. As illustrated in Fig. 5, the gather elastic member 66 is for raising the free portion 64 by an elastic contraction force in a product state.

A fixing structure for the gather elastic member 66 and the gather sheet 65 is not particularly limited. For example, as in the example illustrated in Figs. 5 and 6, a structure can be adopted in which the gather elastic member 66 is bonded and fixed to the gather sheet 65 via a hot melt adhesive at the position of the gather elastic member 66, and facing surfaces of the gather sheet 65 are bonded to each other in a portion other than the fallen portion 63, but the hot melt adhesive is not present at the position of the gather elastic member 66, therefore the gather elastic member 66 and the gather sheet 65 are not bonded to each other, and the facing surfaces of the gather sheet 65 are not bonded to each other at the position having the gather elastic member 66 in the fallen portion 63.

As the gather elastic member 66, a usually used material such as a polystyrene-based rubber, a polyolefin-based rubber, a polyurethane-based rubber, a polyester-based rubber, polyurethane, polyethylene, polystyrene, a styrene-butadiene copolymer, silicone, or polyester can be used. In order to make it difficult to see the gather elastic member 66 from the outside, the gather elastic member 66 preferably has a fineness of 925 dtex or less, a tension of 150 to 350%, and an interval of 7.0 mm or less. Note that as the gather elastic member 66, a tape-like member having a certain width can be used in addition to the elongated member as in the illustrated example.

As the gather sheet 65, various nonwoven fabrics can be used, but in particular, it is preferable to use a nonwoven fabric having a suppressed basis weight and excellent air permeability in order to prevent stuffiness. Furthermore, as the gather sheet 65, in order to prevent urine or the like from passing through the gather sheet 65, to prevent rash, and to enhance feeling (dry feeling) to a skin, it is desirable to use a water repellent nonwoven fabric coated with, for example, a silicone-based, paraffin metal-based, or alkylchromic chloride-based water repellent agent.

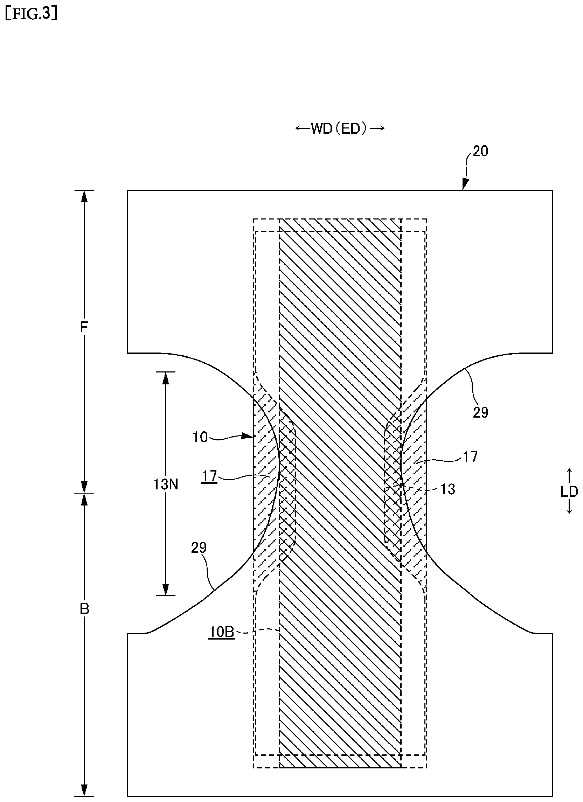

As illustrated in Figs. 3 to 6, a back surface of the inner member 10 is bonded to an inner surface of the outer member 20 with a hot melt adhesive or the like in an inner and outer fixed region 10B (hatched region). The inner and outer fixed region 10B can be appropriately determined and can be substantially the entire inner member 10 in the width direction WD. However, preferably, both end portions in the width direction are not fixed to the outer member 20.

(Structure example of outer member)

The outer member 20 includes at least a lower torso portion T of the front body F and a lower torso portion T of the back body B, and further includes an intermediate portion L which is a range in the front-back direction between the lower torso portion T of the front body F and the lower torso portion T of the back body B in the illustrated example. The outer member 20 may have a side edge located closer to the center in the width direction than a side edge of the inner member 10 at a crotch portion as in the illustrated example, or may have a side edge located outward in the width direction.

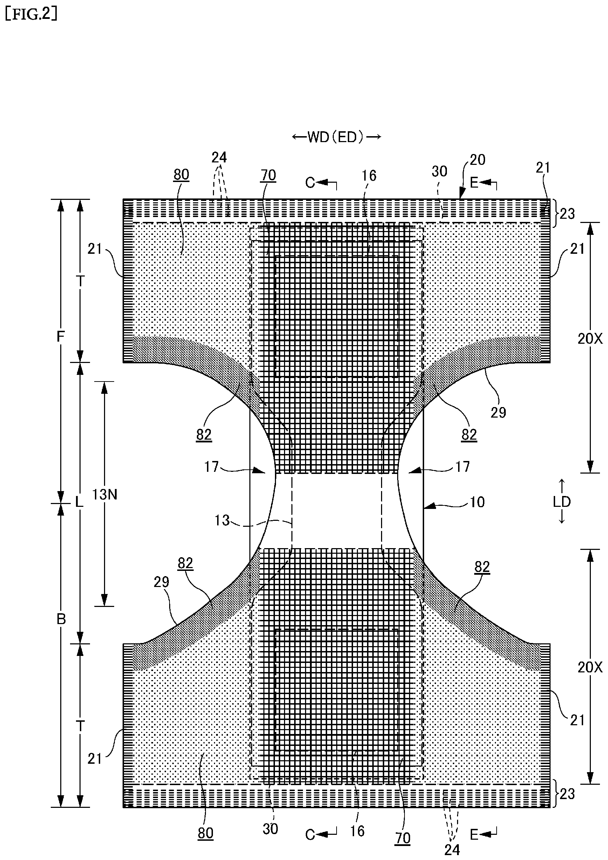

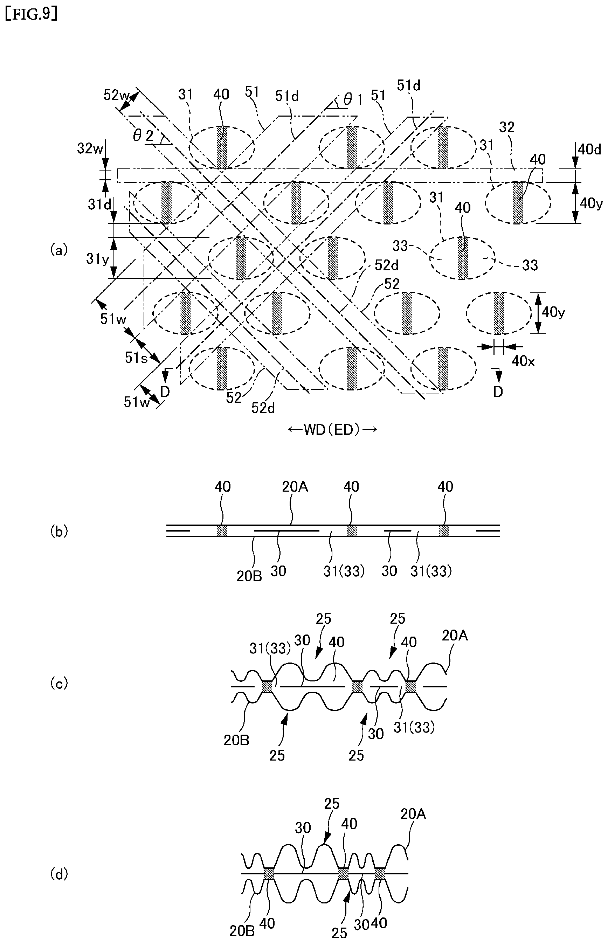

Except for an intermediate of the intermediate portion L in the front-back direction and a waist end portion 23, as illustrated in Figs. 2 and 4 to 6, the outer member 20 in the illustrated example has the elastic sheet 30 interposed between the first sheet layer 20A and the second sheet layer 20B, and as illustrated in Fig. 9 and the like, has an elastic sheet stretchable structure 20X in which the first sheet layer 20A and the second sheet layer 20B are bonded to each other through bonding holes 31 penetrating the elastic sheet 30 with a large number of sheet bonding portions 40 arranged at intervals. A region having the elastic sheet stretchable structure includes a stretchable region that is contracted in the width direction by contraction of the elastic sheet 30 and is extensible in the width direction (that is, the stretchable direction ED is the width direction WD of the diaper).

The planar shape of the outer member 20 is formed by a recessed leg periphery line 29 such that each of both side edges of the intermediate portion L in the width direction forms a leg opening, and has a shape similar to an hourglass as a whole. The outer member 20 may be formed for each of the front body F and the back body B such that the outer members 20 for the front body F and the back body B are separated from each other in the front-back direction LD of the diaper at a crotch portion.

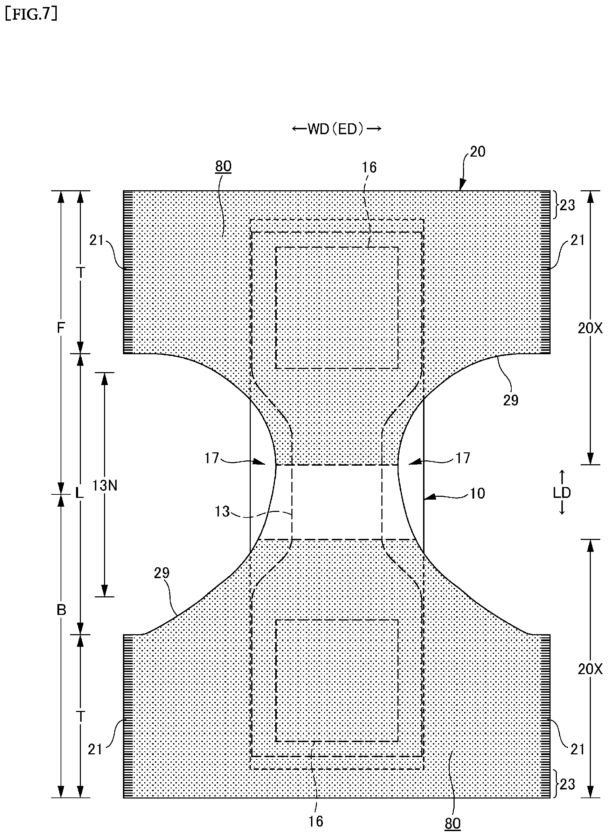

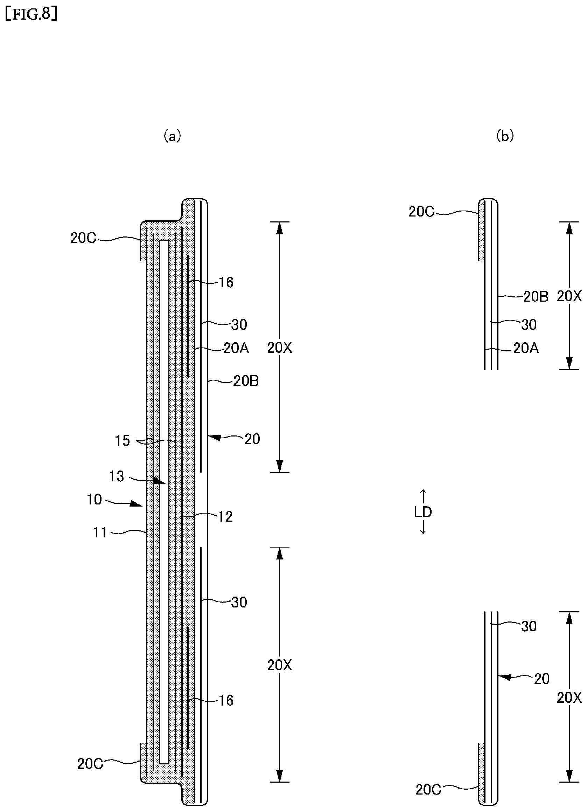

In the form illustrated in Figs. 1 and 2, in order to strengthen tightening of the waist end portion 23, the elastic sheet stretchable structure 20X is not disposed in the waist end portion 23, and a stretchable structure by a conventional elongated waist portion elastic member 24 is disposed. However, as illustrated in Figs. 7 and 8, the elastic sheet stretchable structure 20X may extend to the waist end portion 23 as necessary. The waist portion elastic member 24 is an elongated elastic member such as a plurality of rubber threads disposed at intervals in the front-back direction LD, and applies a stretching force so as to tighten a lower torso of a body. The waist portion elastic members 24 are not disposed substantially in a bundle at close intervals, but three or more, preferably five or more waist portion elastic members 24 are disposed at about 3 to 8 mm intervals in the front-back direction so as to form a predetermined stretchable zone. A stretch rate of the waist portion elastic member 24 at the time of fixing can be determined appropriately, but can be about 230 to 320% for a normal adult. As the waist portion elastic member 24, a rubber thread is used in the illustrated example, but other elongated stretchable members such as flat rubber may be used. Although not illustrated, by disposing the elastic sheet 30 in the waist end portion 23 and disposing the elongated waist portion elastic member 24 at a position overlapping with the elastic sheet 30, a stretchable structure by both the elastic members can be obtained. In the illustrated embodiment, the elongated elastic member extending along the leg opening is not disposed at an edge portion of the leg opening in the outer member 20. However, the elongated elastic member can be disposed at a position overlapping with the elastic sheet 30 at the edge portion or instead of the elastic sheet 30 at the edge portion.

Although not illustrated, appropriate modifications are also possible. For example, another form may be a form in which the elastic sheet stretchable structure 20X is not disposed in the intermediate portion L between the lower torso portion T of the front body F and the lower torso portion T of the back body B, a form in which the elastic sheet stretchable structure 20X is continuously disposed in the front-back direction LD from the lower torso portion T of the front body F up to the lower torso portion T of the back body B through the intermediate portion L, or a form in which the elastic sheet stretchable structure 20X is disposed in only either the front body F or the back body B.

(Stretchable region)

A region having the elastic sheet stretchable structure 20X in the outer member 20 has a stretchable region stretchable in the width direction WD. A stretchable region 80 is contracted in the width direction WD by a contraction force of the elastic sheet 30 and is extensible in the width direction WD. More specifically, in a state where the elastic sheet 30 is stretched in the width direction WD, by bonding the first sheet layer 20A and the second sheet layer 20B to each other via the bonding holes 31 of the elastic sheet 30 at intervals in the width direction WD and the front-back direction LD (direction LD orthogonal to the stretchable direction) orthogonal to the width direction WD, and forming a large number of sheet bonding portions 40, the elastic sheet stretchable structure 20X is formed. In addition, in the stretchable region 80, by disposing the sheet bonding portions 40 such that the elastic sheet 30 remains without interruption in the width direction WD, and the first sheet layer 20A and the second sheet layer 20B are contracted by a contraction force of the elastic sheet 30 to form contraction pleats 25, such elasticity can be imparted.

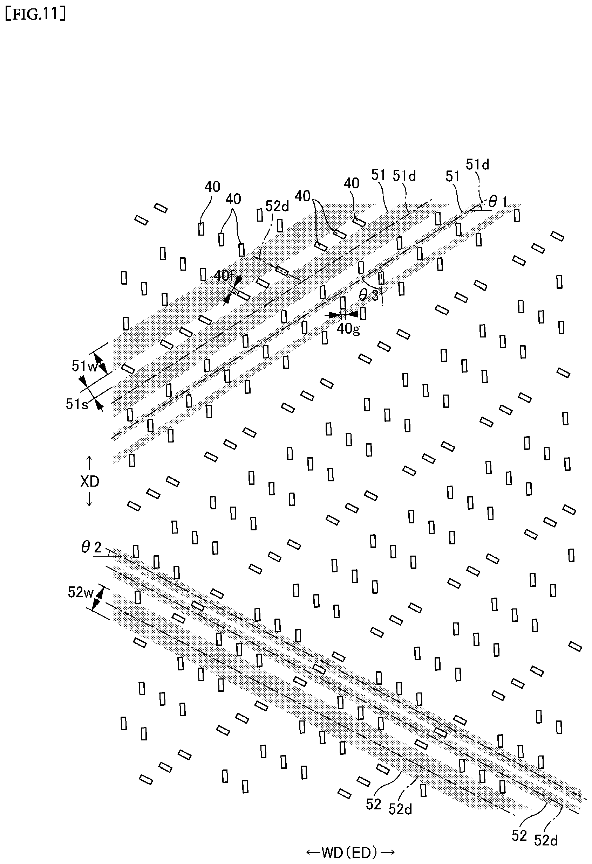

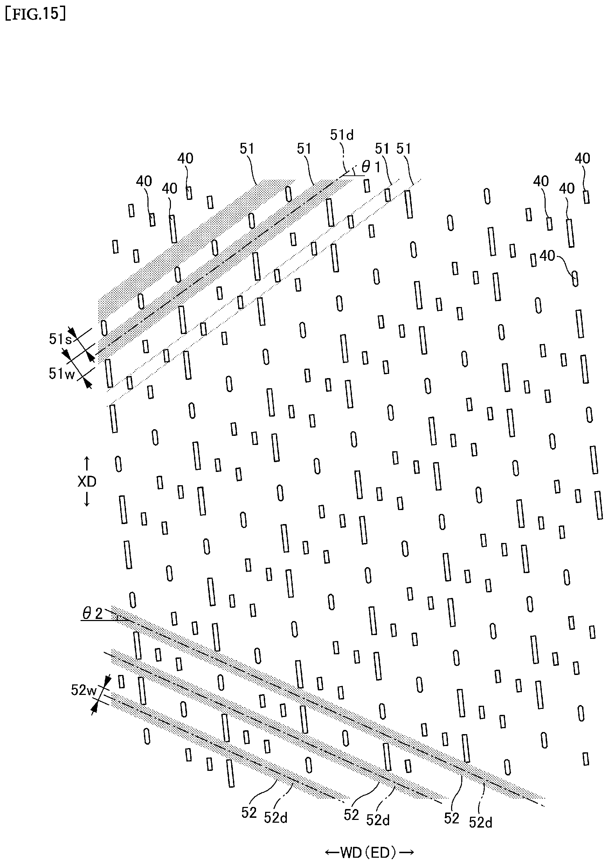

In the stretchable region 80, the elastic sheet 30 may have a portion 32 linearly continuous in the width direction WD as in the example illustrated in Fig. 9, or does not have to have the portion 32 as in the examples illustrated in Figs. 11 and 15.

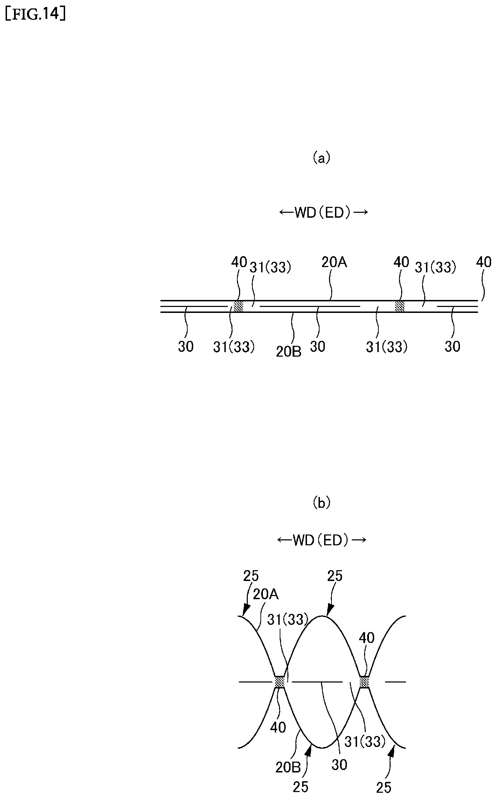

In a natural length state, as illustrated in Figs. 9(d) and 14(b), the stretchable region 80 swells in a direction in which the first sheet layer 20A and the second sheet layer 20B between the sheet bonding portions 40 are separated from each other to form the contraction pleats 25 extending in the front-back direction LD. The contraction pleats 25 are stretched but remain even in a wearing state in which the stretchable region 80 is stretched to some extent in the width direction WD. As in the illustrated embodiment, when the first sheet layer 20A and the second sheet layer 20B are not bonded to the elastic sheet 30 except for at least a portion between the first sheet layer 20A and the second sheet layer 20B in the sheet bonding portions 40, as can be seen from Fig. 9(c) assuming a wearing state and Fig. 9(a) assuming unfolded states of the first sheet layer 20A and the second sheet layer 20B, in these states, edges of the bonding holes 31 in the elastic sheet 30 are separated from outer peripheral edges of the sheet bonding portions 40 in the stretchable direction to open the vent holes 33 (gaps), and air permeability is imparted by the vent holes 33 even when a material of the elastic sheet 30 is an imperforate film or sheet. In particular, when the elastic sheet 30 has the portion 32 linearly continuous in the width direction WD, in a natural length state, the bonding holes 31 are narrowed by further contraction of the elastic sheet 30, and a gap is hardly formed between the bonding holes 31 and the sheet bonding portions 40. When the elastic sheet 30 does not have the portion linearly continuous in the width direction WD, the vent holes 33 remain.

It is desirable that a maximum elongation of the stretchable region 80 in the width direction WD is 190% or more (preferably 200 to 220%). The maximum elongation of the stretchable region 80 is substantially determined by the stretch rate of the elastic sheet 30 at the time of manufacture, but decreases due to factors that inhibit contraction in the width direction WD on the basis of the stretch rate. A main one of such inhibition factors is the ratio of the length L of the sheet bonding portion 40 with respect to a unit length in the width direction WD. The larger this ratio is, the lower the maximum elongation is. In a normal case, the length L of the sheet bonding portion 40 has a correlation with an area ratio of the sheet bonding portions 40. Therefore, the maximum elongation of the stretchable region 80 can be adjusted by the area ratio of the sheet bonding portions 40.

When the elastic sheet 30 has the portion 32 linearly continuous in the width direction WD as in the example illustrated in Fig. 9, a stretch stress of the stretchable region 80 can be adjusted mainly by the sum of an orthogonal size 32w (equal to the interval 31d between the bonding holes) of the portion 32 (see Fig. 9(a)) in which the elastic sheet 30 is linearly continuous in the width direction WD. Meanwhile, as in the examples illustrated in Figs. 11 and 15, when the elastic sheet 30 does not have a portion linearly continuous in the width direction WD, a stretch stress of the stretchable region 80 can be adjusted by an intersection angle formed by a continuous direction of non-bonded bands 51 and 52 in which a portion having no sheet bonding portion is continuous and the stretchable direction ED, and in a normal case, acute intersecting angles θ1 and θ2 formed by the continuous direction of the non-bonded bands 51 and 52 and the stretchable direction ED in an unfolded state are each preferably more than 0 degrees and 45 degrees or less, and particularly preferably 10 to 30 degrees.

The area ratio of the sheet bonding portions 40 and the area of each of the sheet bonding portions 40 in the stretchable region 80 can be appropriately determined, but are preferably within the following ranges in a normal case.

Area of sheet bonding portion 40: 0.14 to 3.5 $mm^2$ (particularly 0.14 to 1.0 $mm^2$)
Area ratio of sheet bonding portions 40: 1.8 to 19.1% (particularly 1.8 to 10.6%)

As described above, the maximum elongation and the stretch stress of the stretchable region 80 can be adjusted by the area of the sheet bonding portion 40. Therefore, as illustrated in Fig. 2, by providing a plurality of regions having different area ratios of the sheet bonding portions 40 in the stretchable region 80, fitting can be changed according to a site. In the form illustrated in Fig. 2, the edge region 82 of the leg opening has a higher area ratio of the sheet bonding portions 40 than the other regions, and therefore has a weak stretch stress and is stretched and contracted flexibly. Of course, as illustrated in Fig. 7, the area ratio of the sheet bonding portions 40 in the stretchable region 80 may be constant.

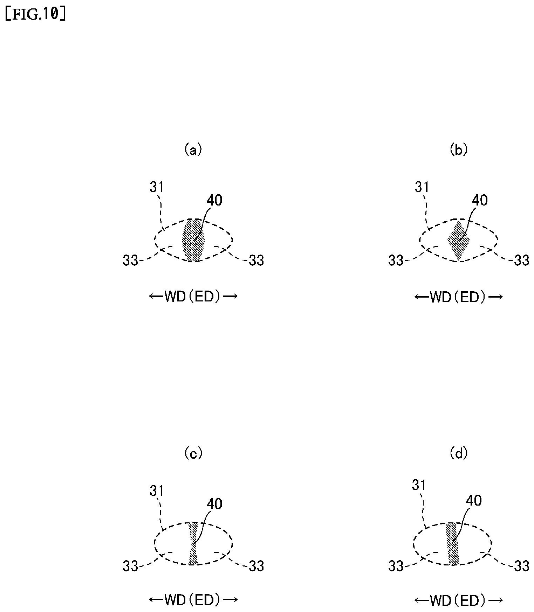

The shape of each of the sheet bonding portions 40 and the bonding holes 31 in a natural length state can be determined appropriately, and can be any shape such as a perfect circle, an ellipse, a polygon such as a triangle, a rectangle (see Figs. 9, 11, and 15), or a rhombus (see Fig. 10(b)), a convex lens shape (see Fig. 10(a)), a concave lens shape (see Fig. 10(c)), a star shape, or a cloud shape. The size of each of the sheet bonding portions 40 is not particularly limited. However, a maximum length thereof 40y (substantially equal to a size 31y of the bonding hole 31 in an orthogonal direction) is preferably 0.5 to 3.0 mm, and particularly prefer ably 0.7 to 1.1 mm. A maximum width thereof 40x is preferably 0.1 to 3.0 mm, and is preferably 0.1 to 1.1 mm particularly in a case of a long shape in a direction XD orthogonal to the stretchable direction.

An arrangement pattern of the sheet bonding portions 40 in the stretchable region 80 is not particularly limited, and any pattern (for example, see Patent Literatures 1 to 8) can be adopted. However, in particular, as in the examples illustrated in Figs. 9, 11, and 15, a non-bonded band in which a portion having no sheet bonding portion is continuous is preferably present in an oblique lattice shape. The illustrated example illustrates a particularly preferable example. In the stretchable region 80, as the non-bonded bands 51 and 52 in which a portion having no sheet bonding portion 40 is continuous in an unfolded state, the first non-bonded band 51 linearly continuous in a first direction 51d intersecting the stretchable direction ED at an acute angle (acute intersecting angle θ1) is repeatedly present at intervals in a direction orthogonal to the first direction 51d. A large number of the sheet bonding portions 40 and the bonding holes 31 are formed at intervals between the adjacent first non-bonded bands 51 in the stretchable region 80. Characteristically, a unit structure including a plurality of the first non-bonded bands 51 having different first widths 51w determined as widths in a direction orthogonal to the first direction 51d is repeatedly present in a direction orthogonal to the first direction 51d in the stretchable region 80.

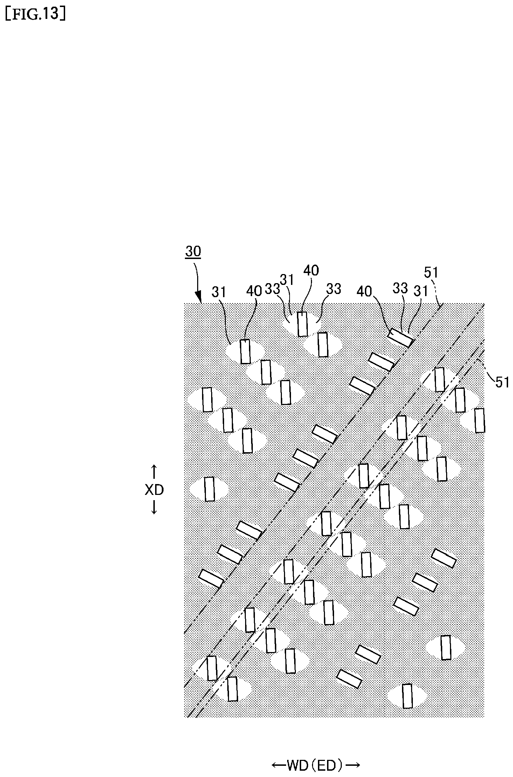

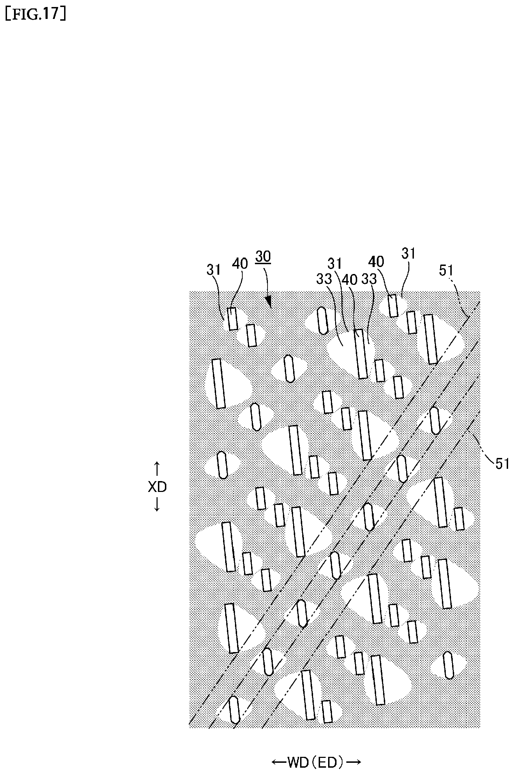

As described above, when the unit structure including the plurality of first non-bonded bands 51 having different first widths 51w is repeatedly present in a direction orthogonal to the first direction 51d in the stretchable region 80, a width change having a similar magnitude relationship is also formed in a continuous portion of the elastic sheet 30 in the first non-bonded bands 51. That is, when the width 51w of the first non-bonded band 51 is narrow, the width of the continuous portion of the internal elastic sheet 30 is also narrow, and when the width 51w of the first non-bonded band 51 is wide, the width of the continuous portion of the internal elastic sheet 30 is also wide. When there is a change in the first width 51w in the continuous portion of the elastic sheet 30 in the first non-bonded bands 51, the stretchable region 80 exhibits a beautiful appearance with an oblique stripe pattern even in a natural length state (see Figs. 13 and 17) or in a wearing state in which the stretchable region 80 is stretched to some extent. That is, in a state where the stretchable region 80 is contracted to some extent, since the size of the contraction pleats 25 in the first non-bonded band 51 changes according to the first width 51w of the first non-bonded band 51, the oblique stripe pattern appears more clearly due to an influence of the contraction pleats 25.

The above-described unit structure is not limited by the degree of magnitude of the width 51w as long as the unit structure includes the plurality of first non-bonded bands 51 having different first widths 51w. However, the first width 51w in the first non-bonded bands 51 is preferably 1.2 to 60 times that of the first non-bonded band 51 having the closest width 51w in a case where the first width 51w is large, and is preferably 0.01 to 0.8 times that of the first non-bonded band 51 having the closest width 51w in a case where the first width 51w is small.

As long as the above-described unit structure includes the plurality of first non-bonded bands 51 having different first widths 51w, the first widths 51w in all the first non-bonded bands 51 may be different from each other, or as illustrated, the first widths 51w in some of the first non-bonded bands 51 may be different from the first widths 51w of the other one or more first non-bonded bands 51.

Even if an oblique stripe pattern in the first direction 51d by the continuous portion of the contraction pleats 25 of the first non-bonded bands 51 appears in the stretchable region 80, when the oblique stripe pattern in another oblique direction is more strongly visually recognized in the same stretchable region 80, the oblique stripe pattern by the continuous portion of the contraction pleats 25 of the first non-bonded bands 51 may be inconspicuous. On the other hand, when the maximum value of the first width 51w in the first non-bonded bands 51 is a maximum value of a width in a direction orthogonal to the continuous direction in all the common non-bonded bands 51 and 52 having different inclination directions, the oblique stripe pattern by the contraction pleats 25 of the first non-bonded bands 51 is more strongly visually recognized in the stretchable region 80, which is preferable. In this case, the maximum value of the first width 51w in the first non-bonded bands 51 can be appropriately determined, but is preferably 1.2 to 60 times that of the first non-bonded band 51 having the closest width 51w. Note that the widths of all the non-bonded bands 51 and 52 including the first non-bonded bands 51 in a direction orthogonal to the continuous direction are not limited, but are preferably 0.02 to 5 mm in a normal case. Needless to say, the widths of the non-bonded bands 51 and 52 in the direction orthogonal to the continuous direction are the first widths 51w in the first non-bonded bands 51, and are equal widths because the first non-bonded bands 51 are linearly continuous portions.

A first interval 51s determined as an interval in a direction orthogonal to the first direction 51d in the adjacent first non-bonded band 51 can be appropriately determined. Therefore, the first interval 51s may be the same as, wider than, or narrower than the first width 51w of the adjacent first non-bonded band 51. As one preferable example, a form can be exemplified in which a maximum value of the first width 51w in the first non-bonded bands 51 in the unit structure is smaller than a maximum value of the first interval 51s. As described above, by forming a wide interval portion in the unit structure, the oblique stripe pattern by the contraction pleats 25 of the first non-bonded bands 51 is more strongly visually recognized. In this case, the maximum value of the first width 51w in the first non-bonded bands 51 can be appropriately determined, but is preferably 0.01 to 9 times the maximum value of the first interval 51s. Note that the interval in all the non-bonded bands 51 and 52 including the first non-bonded bands 51 in a direction orthogonal to the continuous direction is not limited, but is preferably 0.3 to 50 mm in a normal case. Needless to say, the interval in the direction orthogonal to the continuous direction in the non-bonded bands 51 and 52 is the first interval 51s in the first non-bonded bands 51, and is equal to that in the continuous direction.

In the stretchable region 80, as the non-bonded bands 51 and 52, the second non-bonded band 52 linearly continuous in a second direction 52d intersecting the stretchable direction ED at an acute angle (acute intersecting angle θ2) other than the first direction 51d may be repeatedly present at intervals in a direction orthogonal to the second direction 52d, or the second non-bonded band 52 does not have to be present. In a preferred form having the second non-bonded band 52, the non-bonded bands 51 and 52 are formed in an oblique lattice shape in the stretchable region 80, the first non-bonded band 51 is a portion continuous in one direction in the oblique lattice shaped-non-bonded bands 51 and 52, and the second non-bonded band 52 is a portion continuous in another direction in the oblique lattice shaped-non-bonded bands 51 and 52. In this case, the first direction 51d and the second direction 52d have positive and negative inclinations opposite to each other with respect to the stretchable direction ED. Note that, as in the examples illustrated in Figs. 11 and 15, even in a form having no non-bonded bands 51 and 52 continuous in the width direction WD (stretchable direction ED), in an unfolded state of the stretchable region 80, the acute intersecting angle θ1 between the first direction 51d and the stretchable direction ED and the acute intersecting angle θ2 between the second direction 52d and the stretchable direction ED, are 5 to 45 degrees, and particularly 10 to 30 degrees, respectively, whereby the elasticity in the stretchable region 80 can be sufficiently secured.

However, when the oblique stripe pattern in an oblique direction of the second non-bonded bands 52 is more strongly visually recognized in the same stretchable region 80, the oblique stripe pattern by the contraction pleats 25 of the first non-bonded bands 51 may be inconspicuous. Therefore, when the second non-bonded bands 52 are present as in the example illustrated in Fig. 15, it is desirable to arrange the sheet bonding portions 40 such that all the second widths 52w determined as the widths in a direction orthogonal to the second direction in the second non-bonded bands 52 are the same or the second non-bonded bands 52 are not present. As a result, in the stretchable region 80, the oblique stripe pattern by the contraction pleats 25 of the first non-bonded bands 51 is more strongly visually recognized.

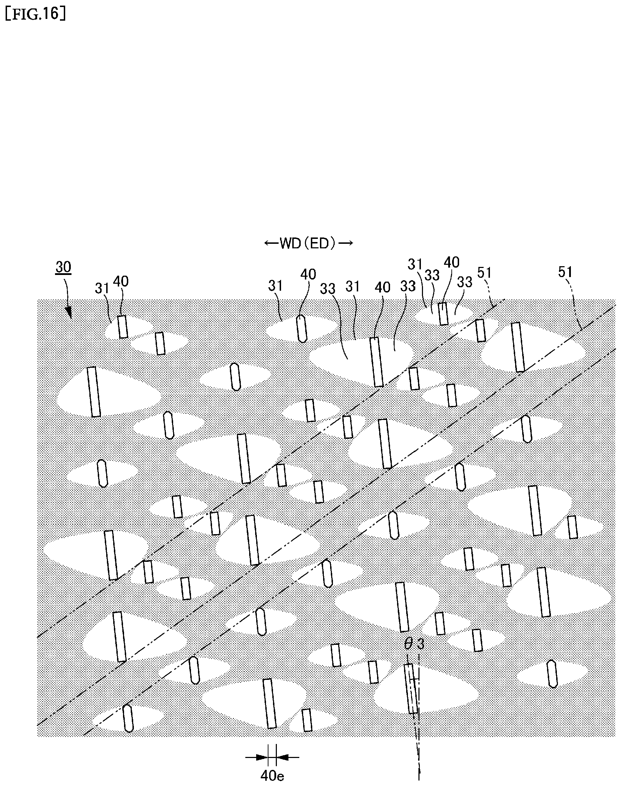

On the other hand, the sheet bonding portions 40 are aligned in the first direction 51d between the adjacent first non-bonded bands 51. In this case, for example, as illustrated in Fig. 16, when all the sheet bonding portions 40 have an elongated shape in which an acute intersecting angle θ3 between a longitudinal direction of the sheet bonding portions 40 and a direction orthogonal to the stretchable direction ED is within 10 degrees and a maximum size 40e in the stretchable direction ED is 0.1 to 0.4 mm, the size of the first non-bonded band 51 in the stretchable direction ED can be secured to be larger, and a decrease in elasticity can be suppressed, which is preferable.

As in the example illustrated in Fig. 11, when the unit structure includes a plurality of wide first non-bonded bands 51 having a maximum first width 51w and a plurality of narrow first non-bonded bands 51 having a first width 51w narrower than the width of the wide first non-bonded band 51 adjacent to each other in a direction orthogonal to the first direction 51d, between the adjacent wide first non-bonded bands 51, the elongated sheet bonding portions 40 in which an acute intersecting angle in a longitudinal direction with respect to the second direction 52d is within 5 degrees and a maximum size 40f in a direction orthogonal to the longitudinal direction is 0.1 to 0.4 mm are preferably aligned at intervals in the first direction 51d. Between the adjacent narrow first non-bonded bands 51, the elongated sheet bonding portions 40 in which the acute intersecting angle θ3 between a longitudinal direction and the first direction 51d is 45 degrees or more and a maximum size 40g in a direction orthogonal to the longitudinal direction is 0.1 to 0.4 mm are preferably aligned at intervals in the first direction 51d. With such shapes and arrangement of the sheet bonding portions 40, the contraction pleats 25 of the first non-bonded bands 51 are particularly visually emphasized with a smaller area of the sheet bonding portion 40.

The number of rows of the sheet bonding portions 40 located between the adjacent non-bonded bands 51 and 52 (the row in the continuous direction of the non-bonded bands 51 and 52) may be one or more. The intervals between the sheet bonding portions 40 in the direction of the row are preferably regular, but all the intervals are not necessarily constant, and some of the intervals may be different.

(Non-stretchable region)

In a region having the elastic sheet stretchable structure 20X in the outer member 20, as illustrated in Fig. 2, a non-stretchable region 70 can be provided on at least one side in the width direction of the stretchable region 80. The non-stretchable region 70 means a region having a maximum elongation of 120% or less in the stretchable direction. The maximum elongation of the non-stretchable region 70 is preferably 110% or less, and more preferably 100%. Arrangement of the stretchable region 80 and the non-stretchable region 70 can be determined appropriately. In a case of the outer member 20 of the underpants-type disposable diaper as in the present example, a portion overlapping with the absorber 13 is a region that does not need to be stretched or contracted. Therefore, as in the illustrated embodiment, a part or the entire part of a portion overlapping with the absorber 13 (it is desirable that almost the entire inner and outer fixed region 10B is included) is preferably set to the non-stretchable region 70. Of course, the non-stretchable region 70 can be provided from a region overlapping with the absorber 13 to a region not overlapping with the absorber 13 located in the width direction WD or the front-back direction LD, or can be provided only in the region not overlapping with the absorber 13. As illustrated in Fig. 7, the non-stretchable region 70 does not have to be provided in the region having the elastic sheet stretchable structure 20X.

The shape of each of the sheet bonding portions 40 in the non-stretchable region 70 is not particularly limited, and can be appropriately selected from shapes similar to those described in the section of the stretchable region 80.

The area ratio of the sheet bonding portions 40 and the area of each of the sheet bonding portions 40 in the non-stretchable region can be appropriately determined, but are preferably within the following ranges in a normal case because the non-stretchable region 70 is not hard due to a small area of each of the sheet bonding portions 40 and a low area ratio of the sheet bonding portions 40.

Area of sheet bonding portion 40: 0.10 to 0.75 mm$^2$ (particularly 0.10 to 0.35 mm$^2$)
Area ratio of sheet bonding portions 40: 4 to 13% (particularly 5 to 10%)

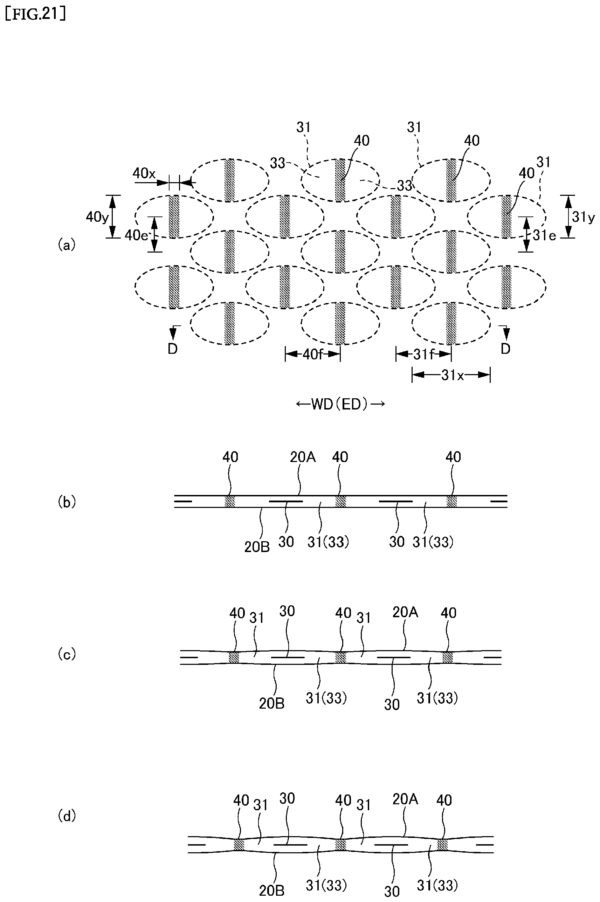

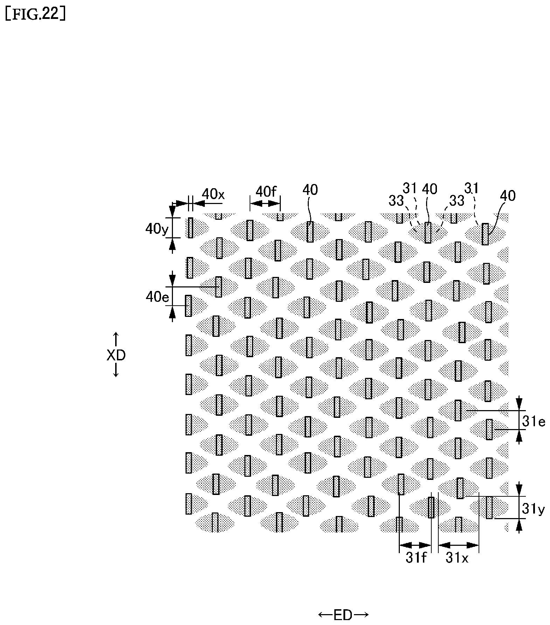

The non-stretchable region 70 can be formed, for example, by densely arranging the sheet bonding portions 40 such that pleats are not formed by contraction of the first sheet layer 20A and the second sheet layer 20B by a contraction force of the elastic sheet 30. Specific example of a technique for forming the non-stretchable region 70 include methods described in Patent Literatures 3 to 6. Figs. 21 and 22 illustrate an example of the non-stretchable region 70 described in Patent Literature 6. In the non-stretchable region 70, the bonding holes 31 are arranged in a zigzag manner in a dense arrangement to some extent or more, and the elastic sheet 30 is continuous in the stretchable direction ED, but there is no portion linearly continuous in the stretchable direction ED due to presence of the bonding holes 31. In this case, as illustrated in Figs. 21 and 22, the vent holes 33 (gaps) are opened with a size such that the size in a natural length state is substantially the same as the size in an unfolded state.

(Bonding structure of sheet bonding portions)

When the first sheet layer 20A and the second sheet layer 20B in the sheet bonding portion 40 are bonded to each other through the bonding holes 31 formed in the elastic sheet 30, it is desirable that the first sheet layer 20A and the second sheet layer 20B are not bonded to the elastic sheet 30 except for at least a portion between the first sheet layer 20A and the second sheet layer 20B in the sheet bonding portion 40.

A means for bonding the first sheet layer 20A and the second sheet layer 20B in the sheet bonding portion 40 to each other is not particularly limited. For example, the first sheet layer 20A and the second sheet layer 20B in the sheet bonding portion 40 may be bonded to each other with a hot melt adhesive or by a bonding means by material welding such as heat sealing or ultrasonic sealing.

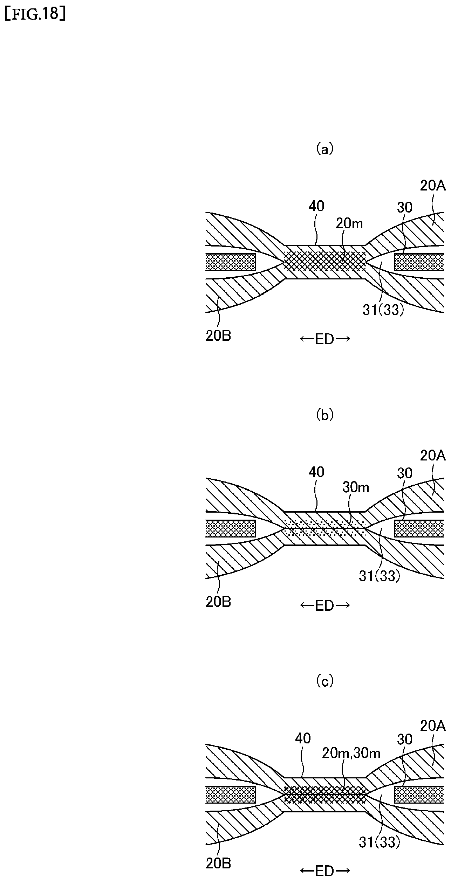

When the first sheet layer 20A and the second sheet layer 20B in the sheet bonding portion 40 are bonded to each other through the bonding holes 31 of the elastic sheet 30, a form in which the sheet bonding portion 40 is formed by material welding may be any one of a first welding form in which the first sheet layer 20A and the second sheet layer 20B are bonded to each other only with a molten and solidified material 20m of a most part or a part of at least one of the first sheet layer 20A and the second sheet layer 20B in the sheet bonding portion 40 (see Fig. 18(a)), a second welding form in which the first sheet layer 20A and the second sheet layer 20B are bonded to each other only with a molten and solidified material 30m of the entire part, a most part, or a part of the elastic sheet 30 in the sheet bonding portion 40 (see Fig. 18(b)), and a third welding form in which both of the first welding form and the second welding form are combined (see Fig. 18(c)), but the second and third welding forms are preferable.

Particularly preferably, the first sheet layer 20A and the second sheet layer 20B are bonded to each other with the molten and solidified material 20m of a part of the first sheet layer 20A and the second sheet layer 20B and the molten and solidified material 30m of the entire part or a most part of the elastic sheet 30 in the sheet bonding portion 40.

As in the first adhesion form and the third adhesion form, when the first sheet layer 20A and the second sheet layer 20B are bonded to each other using the molten and solidified material 20m of a most part or a part of at least one of the first sheet layer 20A and the second sheet layer 20B as an adhesive, it is preferable not to melt a part of the first sheet layer 20A and the second sheet layer 20B because the sheet bonding portion 40 is not cured.

Note that when the first sheet layer 20A and the second sheet layer 20B are formed of a nonwoven fabric, a form in which a part of the first sheet layer 20A and the second sheet layer 20B does not melt includes a form in which cores (including not only a core in a composite fiber but also a center portion of a single component fiber) of all the fibers of the sheet bonding portion 40 remain but portions surrounding the cores (including not only a sheath in a composite fiber but also a portion on a surface layer side of a single component fiber) melt, and a form in which some fibers do not melt at all, but all the remaining fibers melt or cores thereof remain but portions surrounding the cores melt.

When the first sheet layer 20A and the second sheet layer 20B are bonded to each other using the molten and solidified material 30m of the elastic sheet 30 as an adhesive as in the second welding form and the third welding form, peel strength is high. In the second welding form, manufacture is possible by sandwiching the elastic sheet 30 between the first sheet layer 20A and the second sheet layer 20B in which the melting point of at least one of the first sheet layer 20A and the second sheet layer 20B is higher than the melting point of the elastic sheet 30 and the heating temperature at the time of forming the sheet bonding portion 40, pressurizing and heating a site to be the sheet bonding portion 40 to melt only the elastic sheet 30.

Meanwhile, in the third welding form, manufacture is possible by sandwiching the elastic sheet 30 between the first sheet layer 20A and the second sheet layer 20B in which the melting point of at least one of the first sheet layer 20A and the second sheet layer 20B is higher than the melting point of the elastic sheet 30, pressurizing and heating a site to be the sheet bonding portion 40 to melt at least one of the first sheet layer 20A and the second sheet layer 20B and the elastic sheet 30.

From such a viewpoint, the elastic sheet 30 preferably has a melting point of about 80 to 145°C, the first sheet layer 20A and the second sheet layer 20B each have a melting point preferably of about 85 to 190°C, particularly preferably of about 150 to 190°C, and a difference between the melting point of each of the first sheet layer 20A and the second sheet layer 20B and the melting point of the elastic sheet 30 is preferably about 60 to 90°C. The heating temperature is preferably about 100 to 150°C.

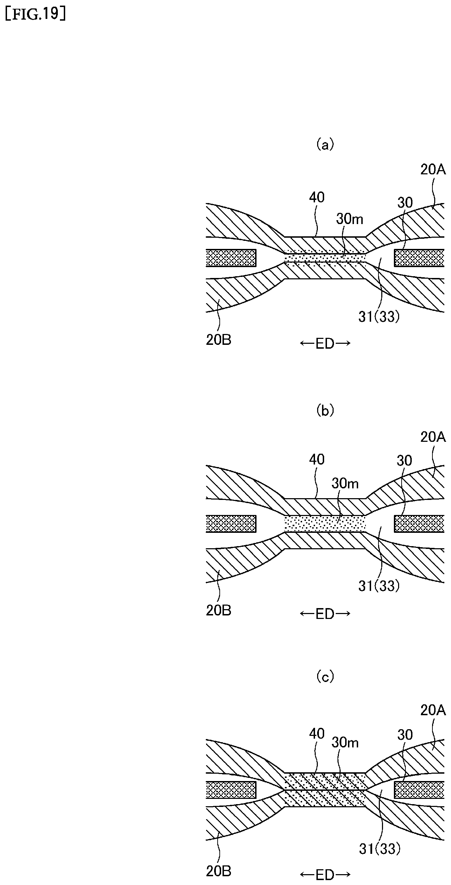

In the second welding form and the third welding form, when the first sheet layer 20A and the second sheet layer 20B are formed of a nonwoven fabric, the molten and solidified material 30m of the elastic sheet 30 may penetrate between fibers over the entire thickness direction of the first sheet layer 20A and the second sheet layer 20B in the sheet bonding portion 40 as illustrated in Fig. 19(c). However, a form in which the molten and solidified material 30m penetrates between fibers up to an intermediate portion in the thickness direction as illustrated in Fig. 19(a), or a form in which the molten and solidified material 30m hardly penetrates between fibers of the first sheet layer 20A and the second sheet layer 20B as illustrated in Fig. 19(b) achieves higher flexibility of the sheet bonding portion 40.

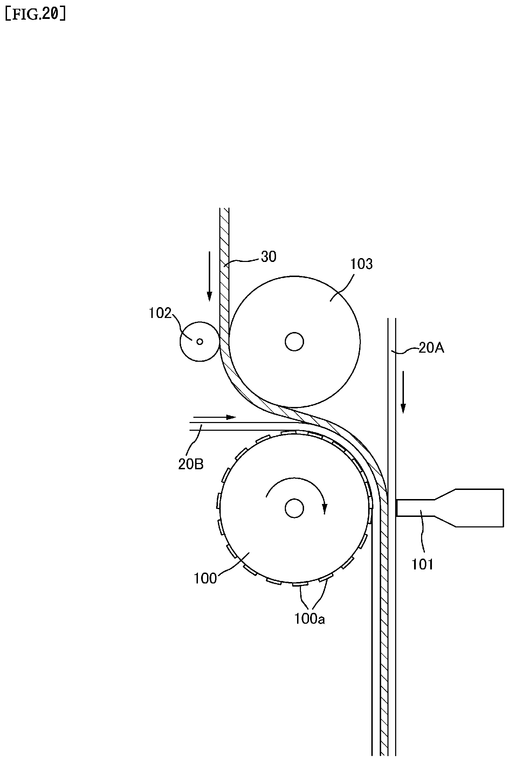

Fig. 20 illustrates an example of an ultrasonic sealing device suitable for forming the second welding form and the third welding form. In this ultrasonic sealing device, for forming the sheet bonding portion 40, the first sheet layer 20A, the elastic sheet 30, and the second sheet layer 20B are fed between an anvil roll 100 having protrusions 100a formed in the pattern of the sheet bonding portions 40 on an outer surface thereof and an ultrasonic horn 101. At this time, for example, by making a feeding transfer speed of the elastic sheet 30 by a feeding drive roll 103 and a nip roll 102 on an upstream side slower than the transfer speed after the anvil roll 100 and the ultrasonic horn 101, the elastic sheet 30 is stretched to a predetermined stretch rate in the MD direction (machine direction, flow direction) in a path from a nip position by the feeding drive roll 103 and the nip roll 102 to a seal position by the anvil roll 100 and the ultrasonic horn 101. The stretch rate of the elastic sheet 30 can be set by selecting a speed difference between the anvil roll 100 and the feeding drive roll 103, and can be set to about 300% to 500%, for example. Reference character 102 represents a nip roll.

The first sheet layer 20A, the elastic sheet 30, and the second sheet layer 20B fed between the anvil roll 100 and the ultrasonic horn 101 are pressurized between the protrusions 100a and the ultrasonic horn 101 and heated by ultrasonic vibration energy of the ultrasonic horn 101 while the first sheet layer 20A, the elastic sheet 30, and the second sheet layer 20B are stacked in this order to melt only the elastic sheet 30 or to melt at least one of the first sheet layer 20A and the second sheet layer 20B and the elastic sheet 30. As a result, the bonding holes 31 are formed in the elastic sheet 30, and simultaneously the first sheet layer 20A and the second sheet layer 20B are bonded to each other through the bonding holes 31. Therefore, in this case, by selecting the sizes, the shapes, a separation interval, an arrangement pattern in a roll length direction and roll circumferential direction, and the like of the protrusions 100a of the anvil roll 100, the area ratio of the sheet bonding portions 40 can be selected.

A reason why the bonding holes 31 are formed is not necessarily clear, but is considered to be that portions corresponding to the protrusions 100a of the anvil roll 100 in the elastic sheet 30 are melted and detached from the surroundings to form holes. At this time, a portion of the elastic sheet 30 between the adjacent bonding holes 31 arranged in the stretchable direction ED is cut from both sides in the stretchable direction by the bonding holes 31 as illustrated in Figs. 9(a), 12, and 13, and loses a support on both sides in a contraction direction. Therefore, the portion of the elastic sheet 30 between the adjacent bonding holes 31 arranged in the stretchable direction ED is contracted until the center side of a direction LD orthogonal to the stretchable direction ED is balanced with the central side of the stretchable direction within a range in which continuity in a direction orthogonal to the contraction direction can be maintained, and the bonding holes 31 expand in the stretchable direction ED.

As a constituent material of the first sheet layer 20A and the second sheet layer 20B, any material can be used without particular limitation, but a material having air permeability is preferable. Therefore, a nonwoven fabric is preferably used from these viewpoints and from a viewpoint of flexibility. In a case where a nonwoven fabric is used, the nonwoven fabric preferably has a basis weight of about 10 to 25 g/m$^2$. A part or the entire part of the first sheet layer 20A and the second sheet layer 20B may be a pair of facing layers obtained by folding back a single material. For example, as in the illustrated embodiment, in the waist end portion 23, by using a constituent material located outside as the second sheet layer 20B, and using a folded-back portion 20C obtained by folding back a waist opening edge thereof to an internal surface side as the first sheet layer 20A, the elastic sheet 30 can be interposed therebetween. Moreover, in a portion other than the waist end portion 23, by using a constituent material located inside as the first sheet layer 20A, and using a constituent material located outside as the second sheet layer 20B, the elastic sheet 30 can be interposed therebetween. Of course, by disposing the constituent material of the first sheet layer 20A and the constituent material of the second sheet layer 20B individually over the entire front-back direction LD without folding back the constituent materials, the elastic sheet 30 can also be interposed between the constituent material of the first sheet layer 20A and the constituent material of the second sheet layer 20B.

The elastic sheet 30 is not particularly limited, and may be a stretchable nonwoven fabric in addition to an elastic film as long as the elastic sheet 30 is a sheet made of a thermoplastic resin having elasticity itself. As the elastic sheet 30, in addition to an imperforate sheet, a sheet having a large number of holes or slits for ventilation can also be used. Particularly, the elastic sheet 30 preferably has a tensile strength of 8 to 25 N/35 mm in the width direction WD (stretchable direction ED, MD direction), a tensile strength of 5 to 20 N/35 mm in the front-back direction LD (direction XD orthogonal to the stretchable direction, CD (cross direction) direction), a tensile elongation of 450 to 1050% in the width direction WD, and a tensile elongation of 450 to 1400% in the front-back direction LD. The thickness of the elastic sheet 30 is not particularly limited, but is preferably about 20 to 40 $\mu$m.

(Color difference)

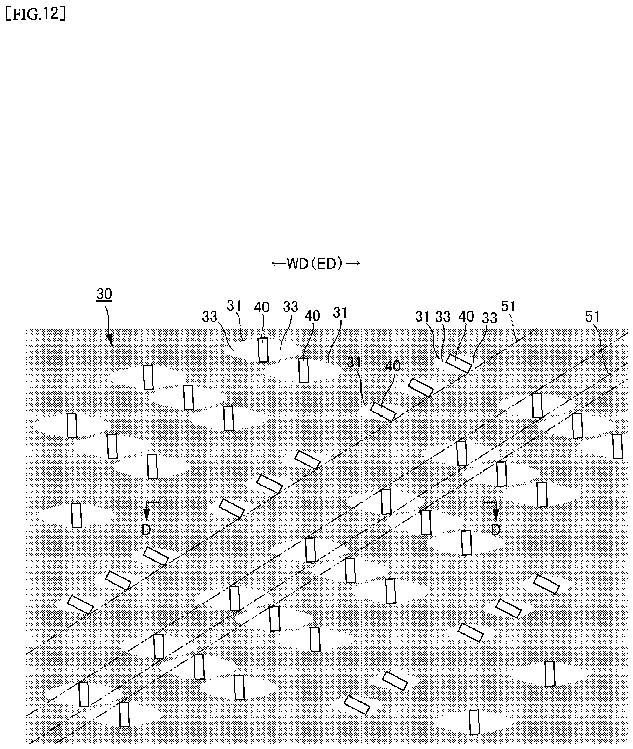

As described above, since the vent holes 33 are formed by separation of edges of the bonding holes 31 from outer peripheral edges of the sheet bonding portions 40 in the stretchable direction ED, the vent holes 33 are deformed by elongation of the elastic sheet 30, and become larger as approaching an unfolded state. Depending on the shape (for example, circular shape) of the sheet bonding portion 40, the vent holes 33 are not necessarily formed because of close contact between the edges of the bonding holes 31 and the outer peripheral edges of the sheet bonding portions 40 in a natural length state. Even in this case, in a state where the elastic sheet 30 is stretched to some extent such as a wearing state, the vent holes 33 are opened at least on both sides of the sheet bonding portions 40 in the stretchable direction ED because the bonding holes 31 are stretched in the stretchable direction ED. Since the vent holes 33 improve air permeability, as illustrated in Figs. 12, 13, 16, and 17 in an emphasized manner, the fact that the vent holes 33 are visually recognized by a user also brings about a functional aesthetic appearance to the product, but there is a possibility that a user may feel that the appearance is not so preferable. Therefore, the color difference $\Delta$E between the color of the outer surface of the elastic sheet 30 and the color of the outer surface of the first sheet layer 20A (lower sheet layer) is 5 or less, and the difference between the color of the outer surface of the elastic sheet 30 and the color of the portion of the outer surface of the first sheet layer 20A seen from the vent holes 33 is small. Therefore, it is difficult to visually recognize the shapes of the vent holes 33 through the second sheet layer 20B (outer sheet layer), which is preferable. The color difference $\Delta$E between the color of the outer surface 30s of the elastic sheet 30 and the color of the outer surface 20s of the first sheet layer 20A is more preferably 3 or less, and particularly preferably 1.5 or less.

A region in which the color difference $\Delta$E between the color of the outer surface 30s of the elastic sheet 30 and the color of the outer surface 20s of the first sheet layer 20A is 5 or less (hereinafter, also referred to as an aesthetic appearance improving region) may be the entire region having the elastic sheet stretchable structure 20X or a partial region such as only an exposed portion exposed to the outer surface. However, it is desirable that the aesthetic appearance improving region is a region including the entire one or plurality of vent holes 33 (preferably bonding holes 31). The aesthetic appearance improving region can be provided only in either one of the stretchable region 80 and the non-stretchable region 70, or can be provided in both of these. The aesthetic appearance improving regions can be provided at a plurality of places at intervals, or the aesthetic appearance improving regions having different color differences can be provided at a plurality of places at intervals or adjacent to each other. For example, the aesthetic appearance improving regions having different color differences can be provided in the stretchable region 80 and the non-stretchable region, or the aesthetic appearance improving regions having different color differences can be provided in the waist end portion 23 region and another region.

If it is only desired to make the shapes of the vent holes 33 difficult to see, the higher the light transmittance of the elastic sheet 30 is, the better. However, within the above-described range of the color difference, when the light transmittance of the elastic sheet 30 is low, for example, when the light transmittance is 40 to 60%, the shapes of the vent holes 33 can be made difficult to see.

When a difference in light transmittance between the first sheet layer 20A (lower sheet layer) and the elastic sheet 30 is large, the shapes of the vent holes 33 are easily visible due to shadow. Therefore, the light transmittance of the first sheet layer 20A is preferably 0.5 to 2.0 times, and particularly preferably 0.8 to 1.6 times the light transmittance of the elastic sheet 30.

If it is only desired to make the shapes of the vent holes 33 difficult to see, the second sheet layer 20B (outer sheet layer) is preferably made of a material having a low light transmittance, for example, a nonwoven fabric having a high basis weight. However, when such a nonwoven fabric is used, texture of an outer surface of a product may be hard, or air permeability may be lowered. Therefore, when the second sheet layer 20B is a flexible and highly air-permeable nonwoven fabric (50% or more, particularly 65 to 80% for light transmittance) in which the constituent fibers have a fineness of 1.0 to 6.0 dtex and a basis weight of 15 to 25 g/m$^2$, and the shapes of the vent holes 33 are made difficult to see due to the above-described color difference, a good performance balance can be obtained.

In order to exhibit good air permeability regardless of a stretched state, as in the stretchable region 80 illustrated in Figs. 11 to 13 and 15 to 17, and the non-stretchable region 70 illustrated in Figs. 21 and 22, the vent holes 33 are preferably opened in a natural length state. However, since this type of product is sold in a natural length state, if the elastic sheet 30 has an opening in the natural length state, an appearance of the product before use may be affected. Therefore, each of the embodiments described above is particularly significant when being applied to a structure in which vent holes 33 are opened in a natural length state.

For various purposes such as adjusting the color difference ΔE between the color of the outer surface 30s of the elastic sheet 30 and the color of the outer surface 20s of the first sheet layer 20A and the light transmittance, and coloring the product, at least one surface of at least one of the elastic sheet 30, the first sheet layer 20A, and the second sheet layer 20B can be colored. A coloring method is not particularly limited, but when a single material is colored in a plurality of colors, coloring can be performed by printing or post dyeing. When the entire material is colored in a single color, printing or post dyeing can be adopted, a technique for mixing a dye or a pigment with a raw material (so-called spun dying, for example, in a case of a nonwoven fabric, a nonwoven fabric is formed with a stock solution colored fiber colored by mixing a dye or a pigment with a stock solution before spinning) can be also adopted.

When such coloring is performed, coloring may be performed only in the aesthetic appearance improving region, or over a wider region including the aesthetic appearance improving region, for example, over the entire material. When a plurality of the aesthetic appearance improving regions is provided, the aesthetic appearances may be colored in different colors.

Of course, as long as the above color difference is satisfied, at least one surface of at least one of the elastic sheet 30, the first sheet layer 20A, and the second sheet layer 20B may be uncolored (that is, the raw material color remains). For example, since the disposable wearing article is a sanitary product, white is often used. Therefore, the outer surface of each of the elastic sheet 30, the first sheet layer 20A, and the second sheet layer 20B preferably has a whiteness of 60 to 80%. In this case, since the whiteness of an outer portion of a hole is low, a difference between the outer portion of the hole and shadow generated at an edge of the hole is reduced, and the holes of the elastic sheet 30 and holes of a perforated nonwoven fabric described later are less likely to be conspicuous while the appearance is white, which is preferred for sanitary products. As is well known, the whiteness of a material can be adjusted by the content of a white pigment such as titanium oxide.

Since the second sheet layer 20B uniformly covers the outer side of the elastic sheet 30, even when the color difference from the elastic sheet 30 is large, an appearance of the vent holes 33 is not directly affected. However, when different colors are stacked, it is difficult to obtain a desired color. Therefore, the difference ΔE between the color of the outer surface of the elastic sheet 30 and the color of the outer surface of the second sheet layer 20B is preferably 5 or less.

When a perforated nonwoven fabric is used for the second sheet layer 20B in order to enhance air permeability of the second sheet layer 20B, the holes of the perforated nonwoven fabric are conspicuous, and there is a possibility that a user may feel that the appearance is not so preferable. On the other hand, when the color difference ΔE between the color of the outer surface of the elastic sheet 30 and the color of the outer surface of the second sheet layer 20B is 5 or less, more preferably 3 or less, or particularly preferably 1.5 or less, the difference between the color of the outer surface of the elastic sheet 30 and the color of the outer surface of the second sheet layer 20B is small. Therefore, the shapes of the holes of the second sheet layer 20B are less likely to be conspicuous. As a result, the present stretchable member has an excellent aesthetic appearance while improving air permeability using a perforated nonwoven fabric for the second sheet layer 20B. Note that a perforated nonwoven fabric can be used for the first sheet layer 20A (lower sheet layer), but it is desirable to use an imperforate nonwoven fabric.

The perforated nonwoven fabric used for the second sheet layer 20B is not particularly limited, and may have holes uniformly formed over the entire second sheet layer 20B, or may partially have a region having no hole.

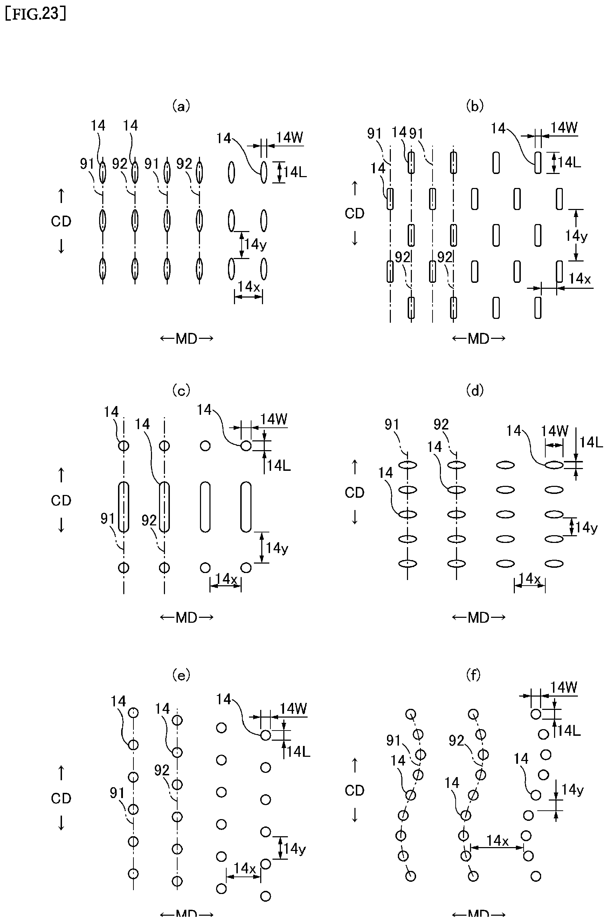

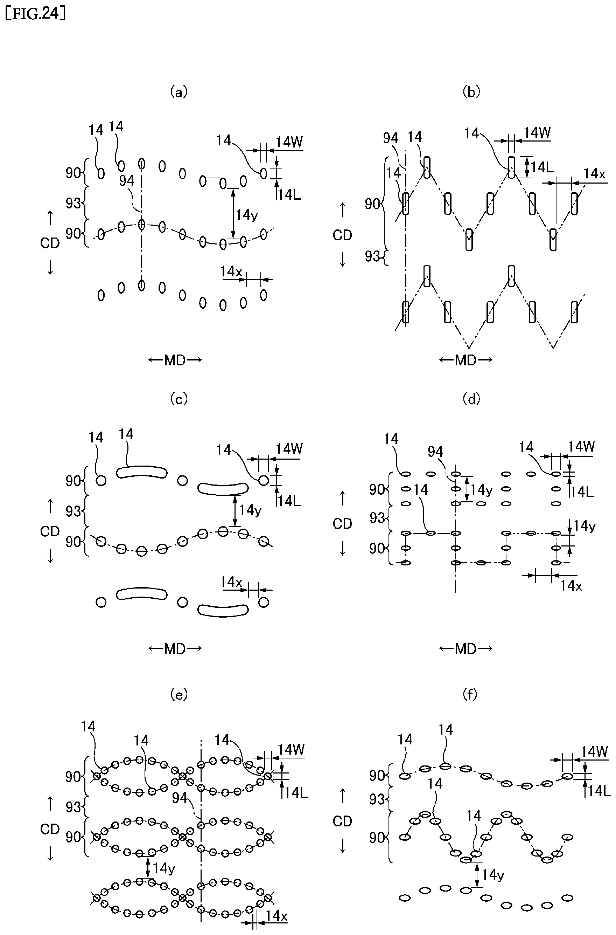

The planar shape (opening shape) of each hole can be appropriately determined. The hole 14 can have, in addition to a long hole shape as illustrated in Figs. 23(b) and 24(b), any shape such as a perfect circle as illustrated in Figs. 23(e), 23(f), and 24(e), an ellipse as illustrated in Figs. 23(a), 23(d), 24(a), 24(d), and 24(f), a polygon such as a triangle, a rectangle, or a rhombus, a star shape, or a cloud shape. As illustrated in Figs. 23(c) and 24(c), the holes 14 having different shapes may be present together. The size of each of the holes 14 is not particularly limited. However, a size 14L in the CD direction (size of the longest portion) is preferably 0.6 to 5.0 mm, and particularly preferably 0.7 to 2.0 mm. A size 14W in the MD direction (size of the longest portion) is preferably 0.6 to 5.0 mm, and particularly preferably 0.6 to 1.2 mm. When the shape of the hole 14 is a shape elongated in one direction (a shape in which the maximum length in one direction is longer than the maximum length in a direction orthogonal thereto) such as a long hole shape, an ellipse, a rectangle, or a rhombus, the size in the MD direction (the size of the longest portion) is preferably 1.2 to 2.5 times the size in the CD direction orthogonal thereto (the size of the longest portion). When the shape of the hole 14 is a shape elongated in one direction, it is desirable that the longitudinal direction of the hole 14 is the MD direction of the nonwoven fabric, but may be the CD direction or an oblique direction inclined thereto. Note that in many cases, the MD direction of the nonwoven fabric is the width direction WD in the outer member 20 of the underpants-type disposable diaper, and is the front-back direction LD in the inner member 200 of the underpants-type disposable diaper, a portion other than a fastening tape in a tape-type disposable diaper, a pad-type disposable diaper, and a sanitary napkin.

It is only required to appropriately determine the area of each of the holes 14 and the area ratio thereof. However, the area is preferably about 0.1 to 2.7 mm$^2$ (particularly 0.1 to 1.0 mm$^2$), and the area ratio is preferably about 1.0 to 15.0% (particularly 5.0 to 10.0%).

A planar arrangement of the holes 14 can be appropriately determined, and may be irregular, a pattern shape, a character shape, or the like, but is preferably a regularly repeated planar arrangement. For example, as illustrated in Figs. 23(a), 23(c), and 23(d), the planar arrangement of all the holes 14 is preferably a matrix in which a row of the holes 14 linearly arranged at predetermined intervals in the CD is repeated at predetermined intervals in the MD direction. In this case, as illustrated in Fig. 23(a), an arrangement can be adopted in which the interval 14x between the holes 14 in the MD direction is shorter than the interval 14y between the holes 14 in the CD direction. In addition, as illustrated in Fig. 23(c), an arrangement can be adopted in which the interval 14x between the holes 14 in the MD direction is almost equal to the interval 14y between the holes 14 in the CD direction, or as illustrated in Fig. 23(d), an arrangement can be adopted in which the interval 14x between the holes 14 in the MD direction is longer than the interval 14y between the holes 14 in the CD direction. As illustrated in Figs. 23(b) and 23(e), an arrangement can be adopted in which rows 91 and 92 of the holes linearly arranged at predetermined intervals in the CD direction are arranged at intervals in the MD direction so as to be shifted in the CD direction. The example illustrated in Fig. 23(b) is a so-called staggered (hexagonal lattice) arrangement in which the arrangement of the holes 14 is alternate in the adjacent hole rows 91 and 92. As illustrated in Fig. 23(f), as long as there is a portion continuous in the CD direction between the adjacent hole rows 91 and 92, an arrangement in which the holes 14 are arranged in a wavy line shape having a center line in the orthogonal direction is also included in the arrangement in which the holes 14 arranged at intervals in the direction orthogonal to the MD direction are arranged at intervals in the MD direction.

The size and shape of the hole 14 in the first hole row 91 and the size and shape of the hole 14 in the second hole row 92 may be the same as each other, but more preferably, at least one of the size and shape is different therebetween.

The CD direction interval 14y between the holes 14 and the MD direction interval 14x between the holes 14 may be constant or may change. These can be determined appropriately. However, in consideration of air permeability, it is desirable that the CD direction interval 14y between the holes 14 is within a range of 0.9 to 8.0 mm, particularly within a range of 1.0 to 3.0 mm, and it is desirable that the MD direction interval 14x between the holes is within a range of 2.0 to 10 mm, particularly within a range of 3.0 to 5.0 mm. In particular, a case where the row of the holes 14 arranged in the CD direction at the CD direction interval 14y narrower than the CD direction size 14L of the hole 14 is repeated at predetermined intervals in the MD direction, and the MD direction interval 14x is wider than the CD direction size 14L of the hole 14 (furthermore, the MD direction interval 14x is more preferably three times or more the MD size 14W of the hole 14) is preferable because softness and bulkiness are not impaired while air permeability is remarkably improved, and a decrease in tensile strength in the MD direction, which is important at the time of manufacturing, is smaller. In particular, in this case, the shape of the hole 14 is preferably a shape elongated in the CD direction.

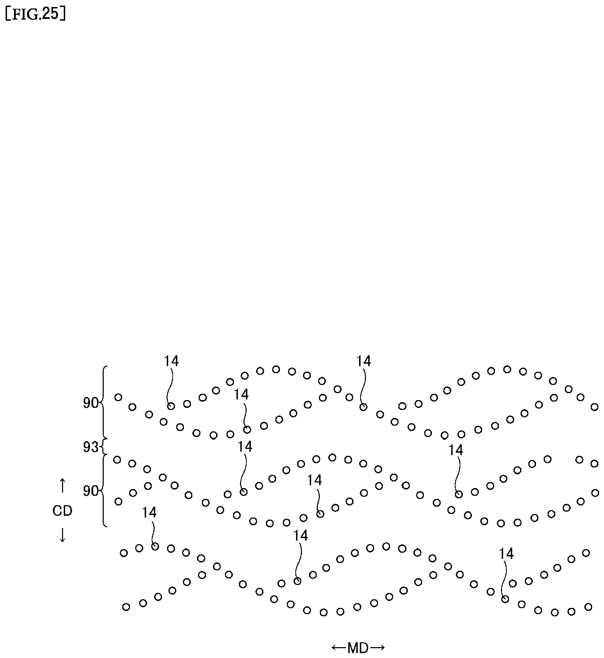

In the planar arrangement of the holes 14, as illustrated in Figs. 24(a) to 24(d) and 24(f), a group 90 of the holes 14 arranged in a single wave shape continuous in the MD direction can be arranged at intervals in the CD direction, and a group 90 of the holes 14 arranged at intervals in a chain shape continuous in the MD direction can be arranged at intervals in the CD direction. The group 90 of the holes 14 arranged in a chain shape is not particularly limited as long as a portion where the holes 14 are annularly arranged is repeated at intervals in the MD direction. For example, the group 90 of the holes 14 arranged so as to form a double wave shape having opposite peaks can be arranged at intervals in the CD direction as illustrated in Fig. 24(e), or the group 90 of the holes 14 can be in a chain shape as illustrated in Fig. 25. Here, "the group 90 of the holes 14 is arranged at intervals in the CD direction" means that an imperforate portion 93 linearly continuous in the MD direction is present between the groups 90 of the holes 14 adjacent to each other in the CD direction.

The arrangement shape in the group 90 of the holes 14 can be appropriately determined as long as the arrangement shape is the above-described wave shape. In addition to a regular shape such as a shape in which peaks and valleys of an arc curve continue repeatedly in a wave shape as illustrated in Fig. 24(a), a triangular wave shape as illustrated in Fig. 24(b), a rectangular wave shape as illustrated in Fig. 24(d), or a sinusoidal shape like the group 90 of the holes 14 in the middle part of Fig. 24(f), a shape in which at least one of the amplitude and the wavelength changes regularly or irregularly, and other irregular shapes can be adopted although not illustrated.

The interval 14x between the holes 14 in the MD direction in the group 90 of the holes 14 may be constant or may change, and can be appropriately determined. In consideration of improvement in air permeability due to the holes 14, it is desirable that the interval 14x is within a range of 2.0 to 10 mm, particularly within a range of 3.0 to 5.0 mm.

As in the example illustrated in Fig. 24(d), the interval 14y between the holes 14 in the CD direction in the row 94 of the holes 14 may change, or may be constant as in the examples illustrated in Figs. 24(a) and 24(b). It is desirable that the interval 14y between the holes 14 in the CD direction in the row 94 of the holes 14 is within a range of 0.9 to 8.0 mm, particularly within a range of 1.0 to 3.0 mm. The interval 14y between the holes 14 in the CD direction in the row 94 of the holes 14 may be longer than or almost equal to the interval 14x between the holes 14 in the MD direction in the group 90 of the holes 14, but it is desirable that the interval 14y is shorter (about 2 to 5 times).

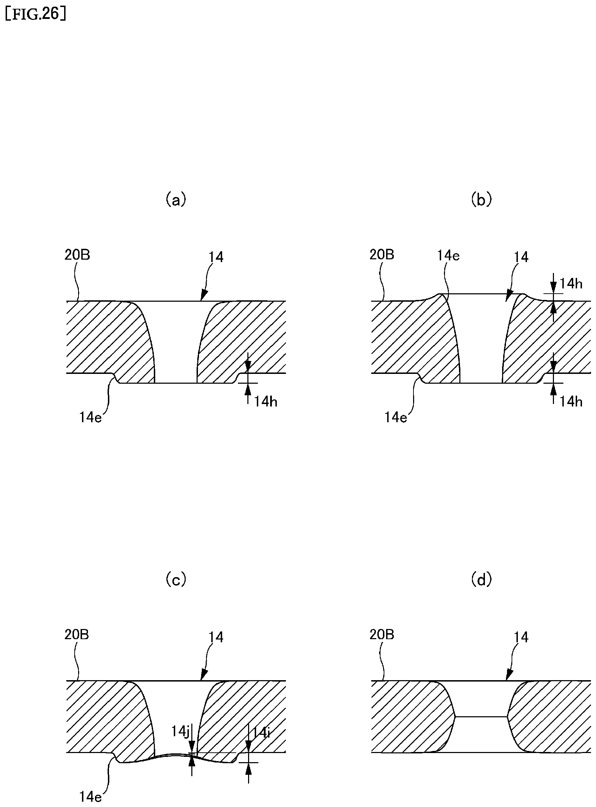

The cross-sectional shape of the hole 14 is not particularly limited. For example, the hole 14 may be a punched hole in which a peripheral edge is formed by a cut end of fibers, or may be a non-punched hole (having a high fiber density at an edge) in which there is almost no cut end of fibers at a peripheral edge of the hole 14 and a pin is inserted between fibers and expanded. As illustrated in Fig. 26(d), the punched hole may be a hole in which the diameter of the hole 14 decreases toward the middle in the thickness direction, or may be a hole in which the diameter of the hole 14 decreases toward one side in the thickness direction although not illustrated.

In the non-punched hole 14, the diameter of the hole 14 decreases from a pin insertion side toward the opposite side. This includes a hole in which the diameter of the hole 14 does not substantially decrease in the middle in the thickness direction in addition to a hole in which the diameter of the hole 14 continuously decreases over the entire nonwoven fabric layer in the thickness direction. Such a non-punched hole includes a hole in which a protruding portion (burr) 14e in which fibers are extruded to the opposite side to a pin insertion side is formed at an edge of the hole 14 on the opposite side to the pin insertion side and the protruding portion 14e is not formed on the pin insertion side as illustrated in Figs. 26(a) and 26(c), and a hole in which the protruding portion 14e in which fibers are extruded to the opposite side to the pin insertion side is formed at an edge of the hole 14 on the opposite side to the pin insertion side and the protruding portion 14e formed by extruding fibers to the pin insertion side is formed on the pin insertion side as illustrated in Fig. 26(b). Furthermore, the former type of hole 14 includes a hole in which a protruding height 14h of the protruding portion 14e is substantially uniform as illustrated in Fig. 26(a) and a hole in which the protruding portion 14e has a facing portion having a highest protruding height 14i and a facing portion facing in a direction orthogonal to the facing portion and having a lowest protruding height 14j as illustrated in Fig. 26(c). It is desirable that the protruding portion 14e is continuous in a circumferential direction of the hole to form a cylindrical shape, but the protruding portions 14e of some or all of the holes 14 may be each formed only in a part of the circumferential direction of the hole 14. The protruding heights 14h, 14i, and 14j (apparent heights in a state where pressure measured using an optical microscope is not applied) are preferably about 0.2 to 1.2 mm. The highest protruding height 14i of the protruding portion 14e is preferably about 1.1 to 1.4 times the lowest protruding height 14j. The protruding height of the protruding portion 14e may change in the circumferential direction of the hole 14.

For example, when the hole 14 having a shape elongated in one direction as illustrated in Figs. 23(a), 23(b), 23(d), and the like is formed by inserting a pin, fibers at an edge of the hole 14 are retracted outward or in the vertical direction, and the protruding portion (burr) 14e in which the protruding height 14i of the facing portion of the hole 14 in the longitudinal direction is higher than the protruding height 14j of a facing portion in a direction orthogonal to the longitudinal direction is formed. The protruding portion 14e of the hole 14 may have a fiber density lower than that of a surrounding portion, but preferably has a fiber density almost equal to or higher than that of the surrounding portion.

(Display sheet)

In the disposable wearing article, as illustrated in Figs. 2, 4, 6, 7, and 8, a display sheet 16 having a display such as a character may be incorporated in at least one of the front body F and the back body B. Such a display sheet 16 is preferably incorporated in the outer member 20 only in consideration of easiness of viewing of a display 27. However, when the outer member 20 is a stretchable member incorporating the elastic sheet 30 as in the present example, incorporating the display sheet 16 between the first sheet layer 20A and the second sheet layer 20B is not preferable because there is a possibility that the display sheet 16 interferes with bonding of the first sheet layer 20A and the second sheet layer 20B. Therefore, the display sheet 16 is preferably interposed between the outer member 20 and the inner member 10 such that display of the display sheet 16 can be seen through the outer member 20. In this case, since the display of the display sheet 16 is seen through the elastic sheet 30, when the vent holes 33 are conspicuous, there is a possibility that the display of the display sheet 16 is hardly seen or a design of the display is impaired. However, when ingenuity such as the above-described color difference is exercised, the display sheet 16 can be easily seen even if the display sheet 16 is interposed between the outer member 20 and the inner member 10.

As a material of the display sheet 16, a resin film is used in terms of being suitable for high-definition printing. When a resin film having high opacity is used, it is necessary to dispose a print portion on an outer surface of the display sheet 16. However, for example, when a resin film having high transparency is used, the print portion can be disposed on an inner surface of the display sheet 16.

The display added to the display sheet 16 is not particularly limited, and a display such as a pattern for decoration (including a picture or a one-point character), a function display such as a use method, a use assistance, or a size, or a mark display such as a manufacturer, a product name, or a characteristic function can be added by printing or the like.

At least one of the inner surface and the outer surface of the display sheet 16 is bonded to a facing surface via a hot melt adhesive.

It is only required to appropriately determine the size of the display sheet 16. However, in a normal case, the display sheet 16 is preferably disposed within a range overlapping with the inner member 10 in the width direction WD at a size equal to or less than the width of the inner member 10. Specifically, when the size of the display sheet 16 is 50 to 100% of the width of the inner member 10, the display sheet 16 is easily bonded to the inner member 10 side at the time of manufacturing, which is preferable.

The display sheet 16 does not have to be disposed.

<Explanation of terms in specification>

The following terms in the specification have the following meanings unless otherwise specified in the specification.

- "Front body" and "back body" mean a front portion and a back portion with the center of an underpants-type disposable wearing article in the front-back direction as a boundary, respectively. A crotch portion means a front-back direction range including the center of a disposable wearing article in the front-back direction. When an absorber has a narrowing portion, the crotch portion means a front-back direction range of the portion having the narrowing portion.
- "Front-back direction " means a direction (longitudinal direction) indicated by a reference character LD in the drawing, "width direction " means a direction (left-right direction) indicated by a reference character WD in the drawing, and the front-back direction and the width direction are orthogonal to each other.
- "MD direction" and "CD direction" mean a flow direction (Machine Direction) in a manufacturing facility and a lateral direction (Cross Direction) orthogonal thereto, one of which is a front-back direction and the other is a width direction depending on a part of a product. The MD direction of a nonwoven fabric is a direction of fiber orientation of the nonwoven fabric. The fiber orientation is a direction in which fibers of a nonwoven fabric are aligned, and can be determined by, for example, a measurement method in accordance with a fiber orientation test method based on zero distance tensile strength by the TAPPI standard method T481 or a simple measurement method for determining a fiber orientation direction based on a tensile strength ratio in a front-back direction and a width direction.
- "Maximum elongation" means a maximum value of elongation in the stretchable direction ED (in other words, an elongation in an unfolded state where the first sheet layer and the second sheet layer are unfolded without contraction or slackness), and represents the length in an unfolded state in terms of a percentage when a natural length is 100%.
- "Area ratio" means the ratio of a target portion with respect to a unit area, and represents a ratio obtained by dividing the total area of a target portion (for example, sheet bonding portions 40, openings of bonding holes 31, and vent holes) in a target region (for example, stretchable region 80 and non-stretchable region 70) by the area of the target region in terms of a percentage. Particularly, "area ratio" in a region having a stretchable structure means an area ratio in an unfolded state. In a form in which many target portions are disposed at intervals, it is desirable to set a target region to a size including 10 or more target portions and to determine the area ratio.
- "Stretch rate" means a value obtained when a natural length is 100%. For example, a stretch rate of 200% is synonymous with an elongation ratio of 2.
- "Basis weight" is measured as follows. A sample or a test piece is predried and then left in a test chamber or an apparatus in a standard state (test location is at a temperature of $23 \pm 1°C$ and a relative humidity of $50 \pm 2\%$) so as to have a constant weight. Predrying refers to causing a sample or a test piece to have a constant weight in an environment of a temperature of 100°C. Note that fibers having an official moisture regain of 0.0% do not have to be predried. A sample of 100 mm × 100 mm in size is cut out from a test piece having a constant weight using a template for sampling (100 mm × 100 mm). The weight of the sample is measured. The weight is multiplied by 100 to calculate the weight per square meter to be used as a basis weight.
- The thickness of an absorber is measured by using a thickness meter (Peacock, dial thickness gauge large type, model J-B (measurement range: 0 to 35 mm) or model K-4 (measurement range: 0 to 50 mm)) manufactured by Ozaki Mfg. Co., Ltd. and making the sample and the thickness meter horizontal.
- "Thickness" other than the above is automatically measured under conditions that a load is $0.098 \text{ N/cm}^2$ and a pressing area is $2 \text{ cm}^2$ using an automatic thickness meter (KES-G5 handy compression measuring program).
- "Tensile strength" and "tensile elongation (breaking elongation)" mean values measured by setting an initial chuck interval (distance between marked lines) to 50 mm and setting a tensile speed to 300 mm/min according to JIS K7127: 1999 "Plastics-Test method for tensile properties-" except that a test piece has a rectangular shape with a width of 35 mm and a length of 80 mm. As a tensile testing machine, for example, AUTOGRAPH AGS-G100N manufactured by SHIMADZU Corporation can be used.

- "Stretch stress" means a tensile stress (N/35 mm) measured at the time of stretch in an elastic region by a tensile test in which an initial chuck interval (distance between marked lines) is set to 50 mm and a tensile speed is set to 300 mm/min according to JIS K7127: 1999 "Plastics-Test method for tensile properties-". The degree of stretch can be determined appropriately depending on a test target. A test piece preferably has a rectangular shape with a width of 35 mm and a length of 80 mm or more. However, if a test piece with a width of 35 mm cannot be cut out, a test piece with a width that can be cut out is prepared, and a value obtained by converting a measured value into a value with a width of 35 mm is used. Even when a target region is small and a sufficient test piece cannot be collected, if the magnitude of a stretch stress is compared, at least comparison is possible appropriately even with small test pieces as long as the test pieces have the same size. As a tensile testing machine, for example, AUTOGRAPH AGS-G100N manufactured by SHIMADZU Corporation can be used.

- An L* value, an a* value, and a b* value of "CIELAB" can be measured as follows in accordance with JIS Z 8781-4 "Part 4: CIE 1976 L*a*b* color space". That is, the first sheet layer (lower sheet layer) and the elastic sheet are each taken out in a single state and used as a sample. Then, for example, using a measuring device such as X-Rite eXact Standard (measurement diameter: 1.5 mm) manufactured by X-Rite Inc., in a state where a white reflection standard (white plate) is disposed on an inner surface of a sample in an unfolded state, three points at appropriate places (an outer surface of a portion having no sheet bonding portion in the first sheet layer (lower sheet layer) and an outer surface of a portion having no bonding hole in the elastic sheet) of the sample are arbitrarily determined, an L* value, an a* value, and a b* value at each of the points are measured, and an average value thereof is taken as a measured value. Note that, when the measurement diameter of the measuring device is larger than an interval between the sheet bonding portions and a bonding interval and it is not possible to measure only the portion having no sheet bonding portion in the first sheet layer (lower sheet layer), or when it is not possible to measure only the portion having no bonding hole in the elastic sheet, a sample is prepared using the same material as the product, and the L* value, the a* value, and the b* value can be measured. Only a large number of portions having no sheet bonding portion in the first sheet layer (lower sheet layer) and only a large number of portions having no bonding hole in the elastic sheet may be cut out, the cut-out portions may be bonded side by side onto a white reflection standard (white plate) so as not to generate a gap, an overlap, or a shadow due to the gap or the overlap, and the L* value, the a* value, and the b* value may be measured.

- The "color difference ΔE" can be calculated by the following formula based on the measured values of the L* value, the a* value, and the b* value.

$$\Delta E = ((\Delta L^*)^2 + (\Delta a^*)^2 + (\Delta b^*)^2)^{1/2}$$

- "Light transmittance" means a light transmittance defined by translucency (JIS method) defined in JIS L 1913: 2010.
- "Whiteness" means ISO 2470: 1999 Paper, board and pulps-Measurement of diffuse blue reflectance factor (ISO brightness) (MOD). In particular, when an object to be measured is a perforated nonwoven fabric, a test piece bundle stacked such that at least a hole of a test piece on an uppermost surface and a hole of a test piece adjacent to a lower side thereof do not overlap with each other is used. Although a sample size is defined in the above definition, the sample size is not limited as long as a sample can be measured with a measuring device. The number of stacked samples is 10. In a case where the whiteness of a perforated nonwoven fabric in a product is measured, when a sample having a size sufficient for measurement cannot be obtained, a sample can be prepared using the same material, and the whiteness thereof can be measured. As a whiteness measuring device, for example, YQ-Z-48B manufactured by Research Institute for Automation of Light Industry Co., Ltd., or a simple spectrophotometer NF333 or a spectral whiteness colorimeter PF-10 manufactured by Nippon Denshoku Industries Co., Ltd. can be used.
- "Unfolded state" means a flatly unfolded state without contraction or slackness.
- The size of each portion means a size not in a natural length state but in an unfolded state unless otherwise specified.
- When environmental conditions in a test and a measurement are not described, the test and the measurement are performed in a test room or an apparatus in a standard state (test location is at a temperature of 23 ± 1°C and a relative humidity of 50 ± 2%).

Industrial Applicability

The present invention can be used for a stretchable member in a general disposable wearing article, for example, various disposable diapers such as a tape-type and pad-type disposable diapers in addition to the underpants-type disposable diaper as in the above example, a sanitary napkin, and disposable wearing articles for swimming and playing in water, as long as the stretchable member has a stretchable region to which an elastic sheet stretchable structure can be applied.

Reference Signs List

| | |
|---|---|
| 10 | Inner member |
| 10B | Inner and outer fixed region |
| 11 | Top sheet |
| 12 | Liquid impervious sheet |
| 13 | Absorber |
| 13N | Narrowing portion |
| 14 | Hole |
| 15 | Wrapping sheet |
| 16 | Display sheet |
| 17 | Non-absorber side portion |
| 20 | Outer member |
| 20A | First sheet layer |
| 20B | Second sheet layer |
| 20C | Folded-back portion |
| 20X | Elastic sheet stretchable structure |
| 21 | Side seal portion |
| 23 | Waist end portion |
| 24 | Waist portion elastic member |
| 25 | Contraction pleats |
| 29 | Leg periphery line |
| 30 | Elastic sheet |
| 31 | Bonding hole |
| 33 | Vent hole |
| 40 | Sheet bonding portion |
| 51, 52 | Non-bonded band |
| 51 | First non-bonded band |
| 51d | First direction |
| 51s | First interval |
| 51w | First width |
| 52 | Second non-bonded band |
| 52d | Second direction |
| 70 | Non-stretchable region |
| 80 | Stretchable region |
| 60 | Rising gather |
| 63 | Fallen portion |
| 64 | Free portion |
| 65 | Gather sheet |
| 66 | Gather elastic member |
| B | Back body |
| ED | Stretchable direction |
| F | Front body |
| L | Intermediate portion |
| LD | Front-back direction |
| T | Lower torso portion |
| WD | Width direction |

**Claims**

**1.** A stretchable member having an elastic sheet stretchable structure, the elastic sheet stretchable structure comprising:

an outer sheet layer having an exposed portion;

a lower sheet layer; and

an elastic sheet interposed between the outer sheet layer and the lower sheet layer, the outer sheet layer and the lower sheet layer being bonded to each other at a large number of sheet bonding portions arranged at intervals through bonding holes penetrating the elastic sheet or via the elastic sheet, wherein

a region having the elastic sheet stretchable structure has a stretchable region that is contracted in a stretchable direction by contraction of the elastic sheet and is extensible in the stretchable direction,

vent holes are opened by separation of edges of the bonding holes from outer peripheral edges of the sheet bonding portions in the stretchable direction at least in an unfolded state,

a color of an outer surface of the elastic sheet and a color of a portion of an outer surface of the lower sheet layer seen from the vent holes are see-through through the outer sheet layer, and

a color difference ΔE between the color of the outer surface of the elastic sheet and the color of the outer surface of the lower sheet layer is 5 or less.

2. The stretchable member according to claim 1, wherein the elastic sheet has a light transmittance of 40 to 60%.

3. The stretchable member according to claim 1 or 2, wherein
the light transmittance of the lower sheet layer is 0.5 to 2.0 times the light transmittance of the elastic sheet.

4. The stretchable member according to any one of claims 1 to 3, wherein

the outer sheet layer is a perforated nonwoven fabric having holes arranged at intervals and penetrating front and back surfaces of the nonwoven fabric, and

a color difference ΔE between a color of an outer surface of the elastic sheet and a color of an outer surface of the outer sheet layer is 5 or less.

5. The stretchable member according to any one of claims 1 to 4, wherein
each of the outer sheet layer, the elastic sheet, and the lower sheet layer has an outer surface having a whiteness of 60 to 80%.

6. The stretchable member according to any one of claims 1 to 5, wherein
the outer sheet layer is a nonwoven fabric having a constituent fiber fineness of 1.0 to 6.0 dtex and a basis weight of 15 to 25 g/m$^2$.

7. The stretchable member according to any one of claims 1 to 6, wherein
the vent holes are opened in the stretchable region in a natural length state.

8. An underpants-type disposable wearing article comprising:

an integrated outer member from a front body to a back body or outer members separately provided on the front body and the back body;

an inner member attached to an intermediate portion of the outer member in a width direction and extending over both front and back sides of a crotch portion;

a side seal portion in which both side portions of the outer member in the front body and both side portions of the outer member in the back body are bonded to each other; and

a waist opening and a pair of left and right leg openings, wherein

the outer member in at least one of the front body and the back body is a stretchable member including the elastic sheet stretchable structure according to any one of claims 1 to 7 over a width direction range corresponding to a portion between the side seal portions in at least a partial range in the front-back direction such that a stretchable direction of the stretchable region is the width direction.

[FIG.1]

[FIG.2]

[FIG.3]

[FIG.4]

(a)

(b)

[FIG.5]

[FIG.6]

[FIG.7]

[FIG.8]

(a)                    (b)

[FIG.9]

(a)

←WD(ED)→

(b)

(c)

(d)

[FIG.10]

(a)

31
40
33--        --33

←WD(ED)→

(b)

31
40
33--        --33

←WD(ED)→

(c)

31
40
33--        --33

←WD(ED)→

(d)

31
40
33--        --33

←WD(ED)→

[FIG.11]

[FIG.12]

←WD(ED)→

30

33 31 40 33 40 31    31 33 40 33    51          51

[FIG.13]

[FIG.14]

(a)

←WD(ED)→

40 31(33)　　20A　　　40 31(33)　　40

30　　31(33)　　20B 30　　31(33)　　30

(b)

←WD(ED)→

25　　　　25

20A

31(33)

40

40

31(33)

20B　　30

25　　　25

[FIG.15]

[FIG.16]

[FIG.17]

[FIG.18]

(a)

(b)

(c)

[FIG.19]

(a)

(b)

(c)

[FIG.20]

[FIG.21]

[FIG.22]

[FIG.23]

(a)

(b)

(c)

(d)

(e)

(f)

[FIG.24]

[FIG.25]

[FIG.26]

(a)

(b)

(c)

(d)

<table>
<tr><td colspan="3" align="center">**INTERNATIONAL SEARCH REPORT**</td><td>International application No.</td></tr>
<tr><td colspan="3"></td><td>PCT/JP2019/045424</td></tr>
</table>

A. CLASSIFICATION OF SUBJECT MATTER
Int.Cl. A61F13/49(2006.01)i
FI: A61F13/49311A

According to International Patent Classification (IPC) or to both national classification and IPC

B. FIELDS SEARCHED

Minimum documentation searched (classification system followed by classification symbols)
Int.Cl. A61F13/49

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2017-225508 A (DAIO PAPER CORPORATION)<br>28.12.2017 (2017-12-28), paragraphs [0035]-[0042],<br>[0049]-[0052], [0066]-[0074], fig. 1-22 | 1-2<br>3-8 |
| Y | JP 2018-164669 A (LIVEDO CORPORATION) 25.10.2018<br>(2018-10-25), paragraph [0049] | 3-8 |
| Y | JP 2017-64225 A (DAIO PAPER CORPORATION)<br>06.04.2017 (2017-04-06), paragraphs [0068]-[0076],<br>fig. 7, 9, 11, 13, 14, 17, 18 | 4-8 |
| Y | JP 2018-51230 A (DAIO PAPER CORPORATION)<br>05.04.2018 (2018-04-05), paragraphs [0066]-[0075],<br>fig. 7(d) | 7-8 |

☐ Further documents are listed in the continuation of Box C.　　☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>08.01.2020 | Date of mailing of the international search report<br>28.01.2020 |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**
Information on patent family members

International application No.

PCT/JP2019/045424

| | | |
|---|---|---|
| JP 2017-225508 A | 28.12.2017 | (Family: none) |
| JP 2018-164669 A | 25.10.2018 | WO 2018/180958 A1<br>paragraph [0050] |
| JP 2017-64225 A | 06.04.2017 | US 2018/0008481 A1<br>paragraphs [0190]-[0198],<br>fig. 23, 25, 27, 29, 30, 32, 33 |
| JP 2018-51230 A | 05.04.2018 | US 2019/0254885 A1<br>paragraphs [0131]-[0141], fig. 7(d) |

Form PCT/ISA/210 (patent family annex) (January 2015)

**EP 4 032 517 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016189932 A
- JP 2015204982 A
- JP 2016140477 A
- JP 2016189931 A
- JP 2016189933 A
- JP 2017064224 A
- JP 2017148169 A
- JP 2017225508 A